## Europäisches Patentamt

## European Patent Office

## Office européen des brevets

(19)

(11) Publication number: **0 183 571**
**A2**

(12)

# EUROPEAN PATENT APPLICATION

(21) Application number: **85308769.0**

(22) Date of filing: **02.12.85**

(51) Int. Cl.⁴: **C 12 N 15/00**
**C 12 N 1/20**
**//(C12N1/20, C12R1:19)**

(30) Priority: **30.11.84 JP 253784/84**

(43) Date of publication of application:
**04.06.86 Bulletin 86/23**

(84) Designated Contracting States:
**CH DE FR GB LI NL**

(71) Applicant: **BANYU SEIYAKU KABUSHIKI KAISHA**
**2-7-8, Nihonbashi-honcho**
**Chuo-ku Tokyo(JP)**

(72) Inventor: **Ishiura, Masahiro**
**Mukoyama apartment 302 2, Mukoyama**
**Myodaiji-cho Okazaki-shi Aichi-ken(JP)**

(72) Inventor: **Ohashi, Hiroshi**
**50-3, Amashiro Higashihamachi**
**Anjo-shi Aichi-ken(JP)**

(72) Inventor: **Okada, Yoshio**
**2-22-28, Tanmachi**
**Minoo-shi Osaka-fu(JP)**

(74) Representative: **Woods, Geoffrey Corlett et al,**
**J.A. KEMP & CO. 14 South Square Gray's Inn**
**London WC1R 5EU(GB)**

(54) **Cosmid vectors.**

(57) Cosmid vectors, each comprising at least three cos of λ-phage or lambdoid phage in tandem, are used to prepare a gene bank by cosmid cloning.

EP 0 183 571 A2

./...

# FIG. 2

FIG. 2

- 1 -

## DESCRIPTION

## COSMID VECTORS

This invention relates to cosmid vectors and their use in the preparation of a gene bank.

Many mammalian, including human, genes are known to have a size of over 20 kilo base pairs (hereinafter designated as Kbp). Since the size of DNA which can be cloned in a conventionally used $\lambda$-phage is 20 Kbp, whole genes can not be cloned in a functional form in this way.

However, DNA of a size of 35-45 Kbp can be cloned using a cosmid vector, a vector having a cohesive end site (hereinafter designated as cos) of $\lambda$-phage. Among the various types of vectors, it is by the use of cosmid vectors that the largest size of DNA can be cloned. However, the application of cosmid vectors has not been widespread due to the difficulty in preparing long genomic DNA fragments, a high background of vectors with no inserts and less effectiveness in preparation.

Ish-Horowicz and Burke have contrived to add directional qualities to cosmid vectors by utilising phosphatase, and succeeded in improving cloning efficiency as well as in reducing the background value of vectors with no inserts [D. Ish-Horowicz and J.F. Burke, Nucleic Acids Res., 9, 2889 (1981)]. The process for preparing a gene bank of genomic DNA according to the Ish-Horowicz and Burke method can be illustrated as follows. A cosmid vector pJB-8, a conventional cosmid vector 5.44 Kbp in size having a restriction endonuclease BamHI, HindIII and SalI cleavage sites with one λ-phage cos, is used.

1. pJB-8 is digested with HindIII to form linear DNA and the HindIII cleavage fragment is inactivated by treating with phosphatase. Cleavage with BamHI provides two DNA fragments·and the large fragment (5.4 Kbp) containing cos is separated by means of agarose gel electrophoresis. The thus obtained DNA fragment (5.4 Kbp) containing cos corresponds to a left arm of λ-phage.

2. pJB-8 is digested with SalI to prepare linear DNA and the SalI fragment is inactivated with phosphatase. Cleavage with BamHI provides two DNA fragments and the small DNA fragment (2.34 kbp) which contains cos is separated by means of agarose gel electrophoresis thereform. The thus obtained DNA fragment (2.34 Kbp) containing cos corresponds to a right arm of λ-phage.

3. Human genomic DNA is partially digested with restriction endonuclease Sau3A or MboI to fractionate 35-40 Kbp DNA fragments. One such fragment is inserted between the left and right arms prepared in 1. and 2. by means of ligase (T4-DNA ligase) to prepare recombinant DNA.

4. The recombinant DNA is converted by means of in vitro packaging to transducing phage particles, which infect E. coli to prepare a human genomic DNA gene bank.

The Ish-Horowicz and Burke method has the following disadvantages:

First, the left and right arms must be prepared individually since pJB-8 has only one cos.

Second, DNA fragments containing cos in the left and right arms must be separated individually by means of agarose gel electrophoresis.

Third, since the length of pJB-8 left side is 5.4 Kbp and that of pJB-8 right side is 2.34 Kbp, i.e. different, the probablility of ligation on 35 - 45 Kbp genomic DNA is different and hence the ratio of the left and right arms should be carefully considered.

Fourth, in the thus prepared gene bank, recombinant DNA can be replicated in E. coli cells as a plasmid and can be isolated as a covalently closed circular plasmid DNA (ccc-DNA) from E. coli cells. However, a genomic DNA gene bank in the form of transducing phage particles can not be prepared from the said recombinant DNA.

The present invention provides cosmid vectors having at least three cos of $\lambda$-phage or lambdoid phage in tandem. Thus, a cosmid vector of the invention comprises at least three cos. The cos are direct repeated sequence.

Preferably, the cosmid vector contains a respective cleavage side for each of two restriction endonucleases such that, on digestion of the cosmid vector with the said restriction endonucleases, the cosmid vector is cleaved into left and right arms wherein one arm comprises at least one cos and the other arm comprises two cos.

The right arm may therefore comprise at least one cos and the left arm comprise two cos. Preferably, the arms are of approximately equal length.

A cosmid vector of the present invention may have three cos in tandem in a molecule. Digestion of the cosmid vector with two kinds of restriction endonuclease provides equimolar amounts of right and left arms of approximately equal length having one or two cos therein. Hence, individual preparation of the right and left arms is not required. Moreover, no agarose gel electrophoretic separation is necessary. Furthermore, since the length of the right and left arms is almost equal, ligation ratio in 35 - 45 Kbp genomic DNA by T4-DNA ligase is expected to be equal. Therefore, equimolar mixture of the right and left arms molecules prepared by digestion with two kind of restriction endonuclease, can directly be ligated with 35 - 45 Kbp genomic DNA to be expected converting half amount of recombinant DNA to transducing phage particles. Accordingly, using a vector of the present invention, 35 - 45 Kbp DNA fragment genomic DNA gene bank can simply and effectively be prepared. Further, since a vector of the present invention has three cos in tandem in a molecule, and hence a recombinant DNA thereof is converted to transducing phage particles by means of in vitro packaging to infect in E. coli, resulting recombinant DNA is replicated a plasmid DNA having two cos in E. coli. At this stage it is an extraneous DNA gene bank of a plasmid DNA, however in case that E. coli, in which λ-phage is lysogenized, is used as a host, lysogenized λ-phage enters lytic cycle by an induction such as heating or ultraviolet irradiation, thus λ-phage components are expressed in E. coli cells and finally E. coli is lysogenized to disperse out the λ-phage particles. Simultaneously, the recombinant DNA in the form of plasmid DNA having two cos in E. coli cells is introduced into a head of λ-phage, then transferred into transducing phage particles by means of in vitro packaging. According to the thus illustrated procedures, extraneous DNA gene bank as transducing pahge particles can be prepared.

In a gene transformation of mammalian gene into cultured cells, a method using chromosome has advantages as compared with a method using plasmid DNA or genomic DNA as follows.

(1) Effectiveness and frequency for DNA transformation are 10 to 100 times higher than as usual, and

(2) Stable transformant clone can be obtained.  [Lewis, W.H., Srinivasan, P.R., Stockoe, N. and Siminovitch, L., Somat. Cell. Genet., 6, 333 (1980)].  These facts may have correlations that DNA in chromosome is closely enveloped by chromation protein to construct chromatin structure, and so is defended from attacking by deoxyribonuclease (DNase).  Alternatively, constructing dense chromatin structure may have resulted easy incorporation into cells.  However only chromosome itself can not be recombined the DNA/and large amount of chromosome required for experimental use can not easily be prepared from cells.  Referring to the structure of λ-phage particles, DNA of λ-phage is enveloped densely with outer envelope protein, and this state resembles to chromosome DNA enveloped with protein.  A method for preparing gene bank of λ-phage particles by recombinant DNA has been established.

Ishiura et al demonstrated transfer of HSV-1TK gene int Ltk$^-$ cells using λ-vector Char b$^n$/4A recombinant (λ-phage particles) integrated with HSV-1TK gene, and found that:

(1) Efficiency of gene integration is $10^{-5}$ and is higher at about one figure as compared with that of DNA itself, and is lower at one figure as compared with that of chromosome. and (2) Size of colony of transforming cell is larger than that obtained from DNA, and many of clones can be kept stable in non-selective medium. [Ishiura, M. et al., Mol. Cell. Biol., 2, 607 (1982)].

Substantial problem on gene bank prepared by λvector is that maximum size of DNA which can be cloned in λ-vector is at maximum 20 Kbp, and hence if size of gene is larger than that, the said gene can not be cloned with functional form.

- 7 -

A cosmid vector having three cos in tandem /of the present invention has advantages that it can clone with 35 - 45 Kbp extraneous DNA and can preparegene bank with transducing λ-phage particles. The present process can exhibit a great possibility for transforming gene in cultured cells.

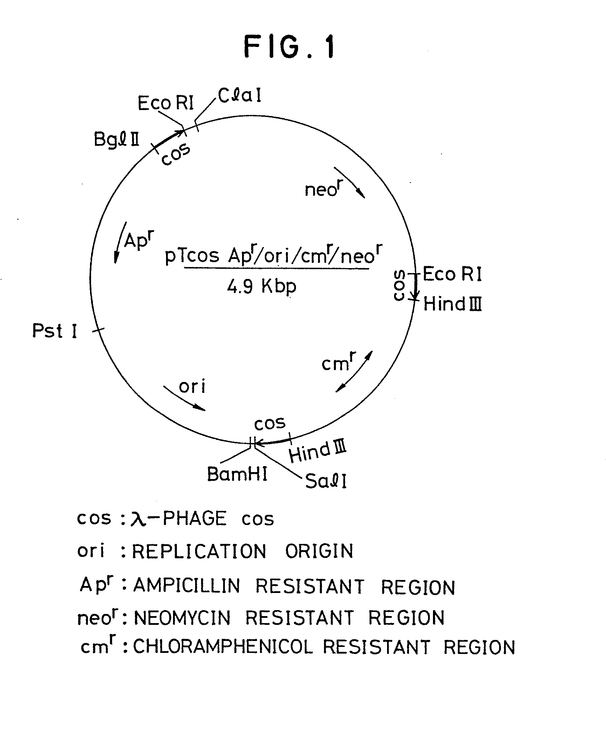

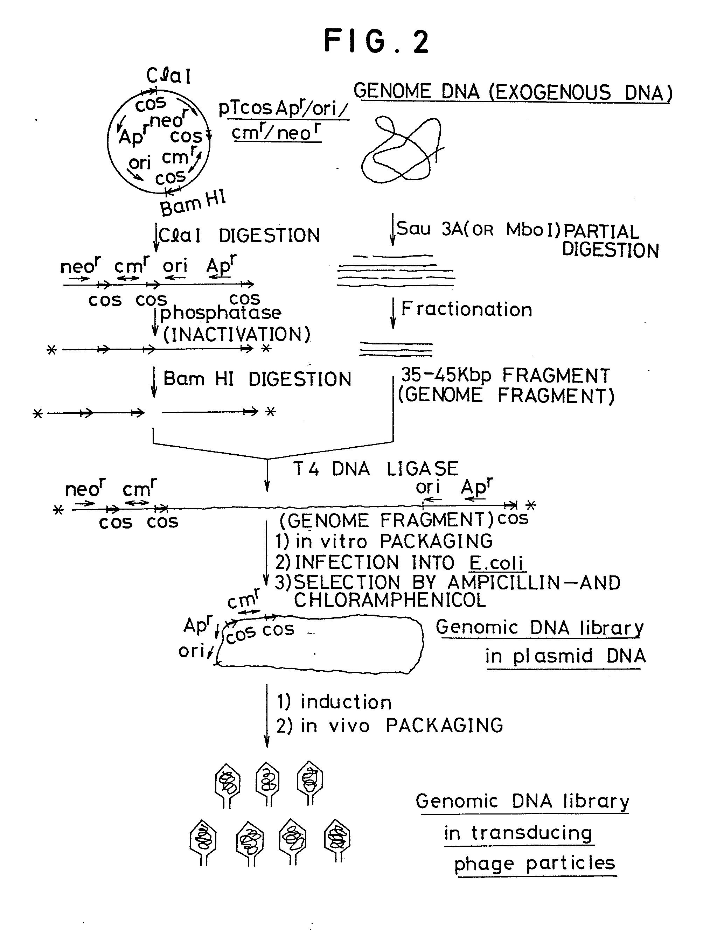

In Fig. 1, a novel cosmid vector of the present invention pTcos $Ap^r$/ori/$cm^r$/$neo^r$ is illustrated. The pTcos $Ap^r$/ori/$cm^r$/$neo^r$ consists of three cos in tandem /which is are originated from cosmid vector pRH(-44) or pLH(+129) and has same direction [Miwa and Matsubara, Gene, 20, 267 (1982)], neomycin resistant gene ($neo^r$) originated from Tn903 [Oka, Sugisaki and Takanami, J. Mol. Biol., 147, 217 (1981)], chloramphenicol resistant gene ($cm^r$) originated from pNT-31 [Tomizawa and Itoh, Proc. Natl. Acad. Sci., U.S.A., 78, 6096 (1981)], ampicillin resistant gene ($Ap^r$) originated from pBR-327 and origin of replication (ori) [Soberon, Cavarrubias and Bolivar, Gene, 9, 287 (1980)]. Size of the said cosmid vector is 4.9 Kbp and is cleaved with restriction endonuclease ClaI and BamHI at one point, respectively. A process for preparing genomic DNA (extraneous DNA) bank using the said vector is illustrated as follows (Fig. 2).

(1) A cosmid vector pTcos $Ap^r$/ori/$cm^r$/$neo^r$ is cleaved with ClaI to prepare linear DNA, then is treated with phosphatase to inactivate ClaI fragment. Further, the fragment is digested with BamHI to prepare right and left arms of the equal length having one or two cos, respectively.

(2) Genomic DNA (extraneous DNA) is partially digested with Sau3A or MboI to prepare 35 - 45 Kbp DNA fragment.

(3) Genomic DNA fragment (35 - 45 Kbp) is inserted into between the left and right arms prepared in (1) hereinabove by means of T4-DNA ligase to prepare recombinant DNA.

(4) The recombinant DNA is converted to transducing phage particles by means of in vitro packaging, which is infected into λ-phage lysogenic E. coli cells.

(5) Since the said lysogenic DNA contains $Ap^r$, $cm^r$ and ori originated from vector, E. coli cells which include plasmid of recombinant DNA having two cos can be selectively grown by selection with ampicillin or chloramphenicol.

(6) A transformant (E. coli) is subjected to induction such as heating or ultraviolet irradiation to transfer the lysogenic λ-phage into a lytic cycle, thereby the recombinant DNA having two cos can be converted to transducing phage particles by means of in vivo packaging. The said transducing phage particles include the recombinant DNA comprising a cos, $Ap^r$ , ori and extraneous DNA.

As illustrated, a genomic DNA (extraneous DNA) gene bank can be prepared as a transducing pahge particles.

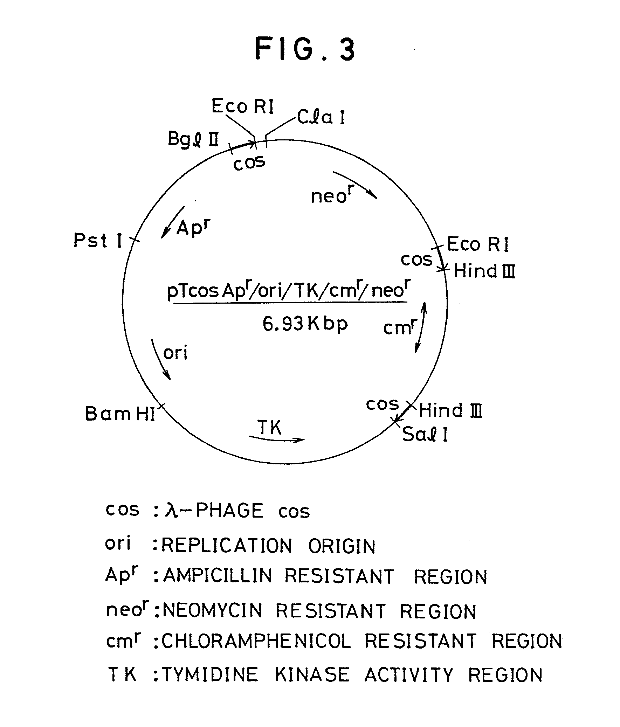

We have also prepared cosmid vector, pTcos $Ap^r$/ori/ TK/$cm^r$/$neo^r$ which has specific marker for mammalian cells and is able to transfer the gene in mammalian cells (Fig. 3). /bank The pTcos $Ap^r$/ori/TK/$cm^r$/$neo^r$ consists of three cos with identical direction originated from pRH(-44) or pLH(+129), $neo^r$ from Tn903, $cm^r$ from pNT-31, $Ap^r$ and ori from pBR-327 and TK gene from pTK-4. [Ishiura, M. et al., Mol. Cell. Biol. 2, 607 (1982)].

Genomic gene bank can be prepared by the said cosmid vector as like the above mentioned pTcos $Ap^r$/ori/$cm^r$/$neo^r$. Namely, three cos in tandem is cleaved with ClaI and BamHI to prepare equimolar amount of the right and left arms        having one or two cos, respectively. The recombinant DNA can be prepared by inserting 35 - 45 Kbp genomic DNA in <u>Bam</u>HI site thereof. The said recombinant DNA can be converted to transducing phage particles by means of in vitro packaging, then it can be transferred into <u>E. coli</u> cells, in which λ-phage is lysogenized, which can be selected by ampicillin or chloramphenicol treatment. The resulted transformant (<u>E. coli</u>) is subjected to induction to transfer the recombinant DNA having two cos in transducing phage particles by means of in vivo packaging, then the gene bank is prepared. Further since the said transducing phage particles have a marker gene(TK), a judgment on tranfer of recombinant DNA originated from vector can advantageously be made at a cultured cell level, by checking a thymidine kinase (TK) activity at transferring a genomic gene bank cells to cultured cells.

The present invention is, therefore, the above illustrated novel cosmid vector and process for preparing a gene bank using the same.

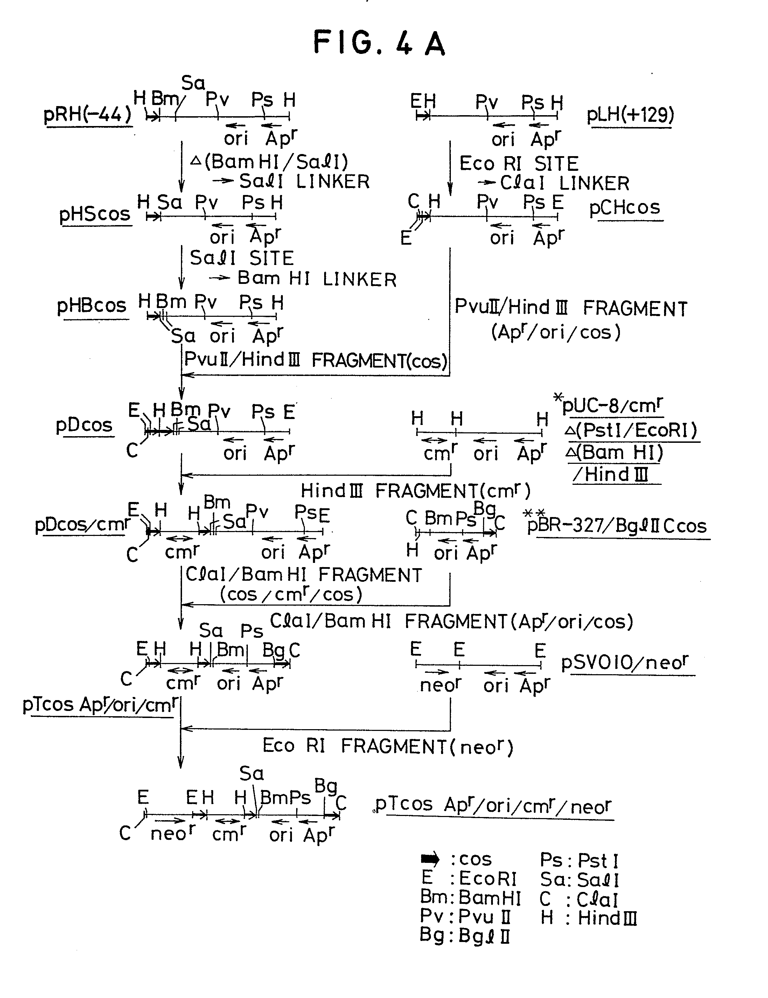

We have also prepared a cosmid vector, pDcos/cm$^r$ (Fig. 4A). The pDcos/cm$^r$ consists of two cos in tandem originated from pRH(-44) and pLH (+129), cm$^r$ from pNT-31, Ap$^r$ and ori from pBR-322. The pDcos/cm$^r$ was digested with two kinds of restriction endonuclease, BamHI, claI to right and left arms having one cos, respectively. The recombinant DNA can be prepared by inserting 35 - 45 kbp genomic DNA in BamHI site thereof. The said recombinant DNA can be converted to transducing phage particles by means of in vitro packing, then it canbe transferred into E. coli, which can be selected by ampicillin. At this stage it is an extraneous DNA gene bank of a plasmid DNA.

Example of cos used in the present invention is orginated from λ-phage or lambdoid phage. The cos originated from λ-phage is preferable. In order to prepare gene bank of transducing phage particles with cosmid vector of the present invention, it is sufficient to include three cos in tandem direction within a vector molecule, however the number of the cos more than three is not limited.

A vector of the present invention is, at first, digested with two kinds of restriction endonucleases. Examples of a combination of the said restriction endonuclease are PvuII and BamHI, PvuII and BglII, ClaI and BamHI or ClaI and BglII. Other combinations thereof can be exist and are not limited.

In order to check an insertion of DNA fragment originated from the vector , a selection by applying drug resistance can generally be performed. Therefore, vector of the present invention should preferably contain DNA fragment which express drug resistance. Example of drug resistance is generally antibiotic resistance such as ampicillin resistance, tetracycline resistance, neomycin (kanamycin) resistance or chloramphenicol resistance.

In pTcos $Ap^r$/ori/$cm^r$/$neo^r$ and pTcos $Ap^r$/ori/TK/$cm^r$/$neo^r$, ampicillin resistance gene, chloramphenicol resistance gene and neomycin resistance gene exist therein. A pDcos/$cm^r$ has ampicillin resistance gene, chloramphenicol resistance gene.

Preparing a genomic DNA (extraneous DNA) gene bank using a vector of the present invention and transferring the gene into cultured mammalian cells, a DNA fragment, which express specific physiological function for determining an insertion of DNA fragment from the original vector, is preferably contained in the vector of the present invention. Examples of gene expressing specific physiological function are thymidine kinase (TK) gene [Wigler, M., et al., Cell, 14, 725 (1978)], adenine phosphoribosyltransferase (APRT) gene [Wigler, M. et al., Proc. Natl. Acad. Sci., U.S.A., 76, 1373 (1979)], hypoxanthine guanine phosphoribosyltransferase (HGPRT) gene [Graf, L.H. Jr., et al., Somat. Cell. Genet., 5, 1031 (1979)] and xanthine guanine phosphoribosyltransferase (XGPRT or gpt) gene [Berg, P. et al., Mol. Cell. Biol., 1, 449 (1981)]. The novel cosmid vector of the present invention pTcos $Ap^r$/ori/TK/$cm^r$/$neo^r$ includes TK gene.

Further, a vector of the present invention preferably contains a gene fragment having replication function originated from ColEI.

Examples of the extraneous DNA used in the present invention are DNA originated from microorganisms such as bacteria, fungi or yeast, higher animals and plants, and phages.

Further, restriction endonuclease, ligase, or an enzyme which catalyses to prepare blunt end in each distal of DNA frangment for ligasing each non-complimentary end of DNA and ligase thereof can be commercially available, and application thereof have widely known. [Molecular Cloning, Cold Spring Harbor Laboratory, Maniatis, T. et al., (1982)].

Preparing the geneomic DNA (extraneous DNA) gene bank using a vector of the present invention, specific gene, which can express useful substances, can effectively be found.

Examples of the specific gene are DNA originated from microorganisms such as bacteria, fungi or yeast, higher animals and plants, or phages. Examples of useful substances are hormons, enzymes or enzymes for synthesis for various hormons, enzymes for synthesizing antibiotics and amino acids.

Following examples illustrate the present invention but are not construed as limiting.

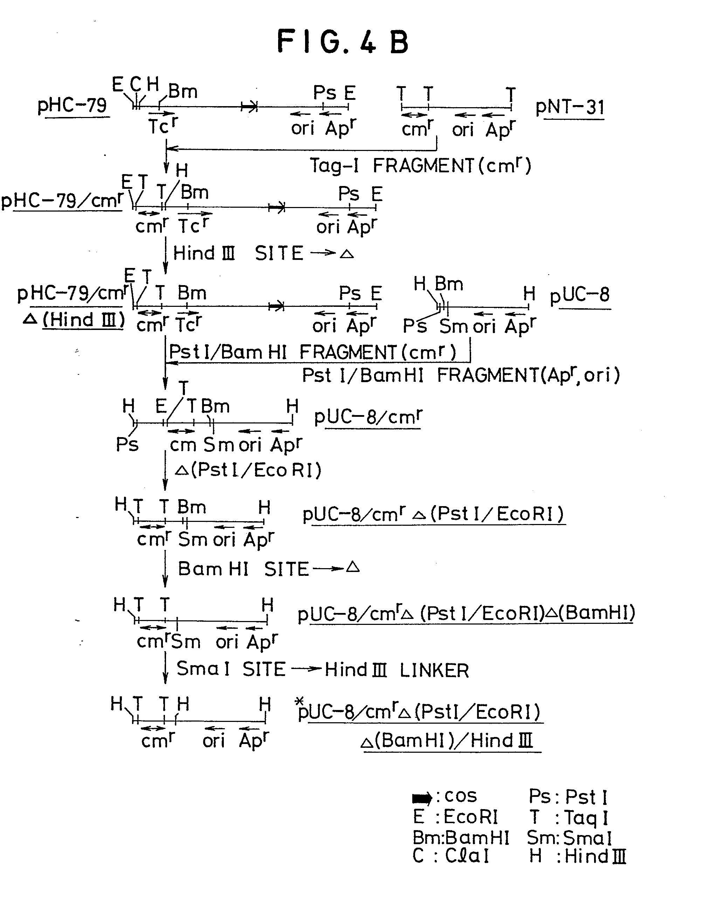

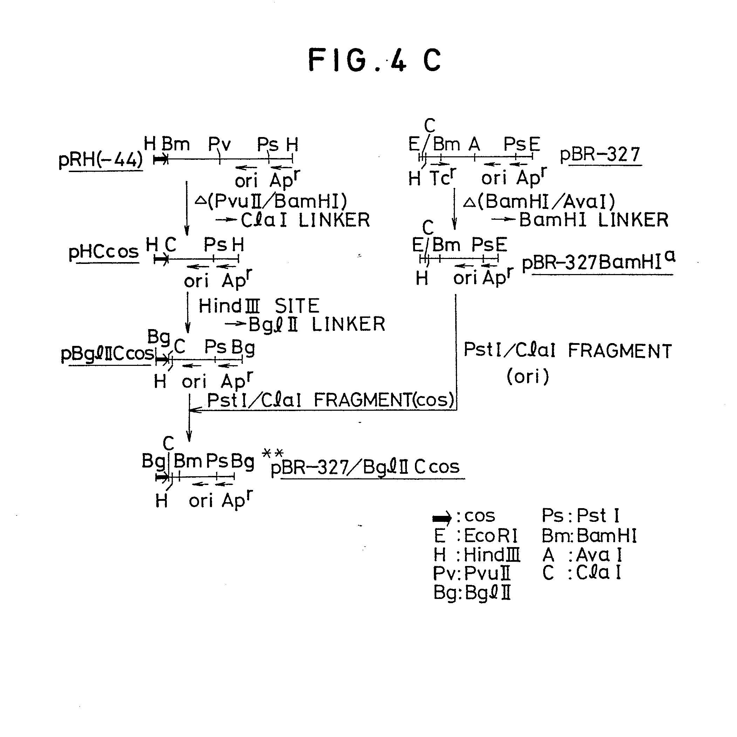

Example 1. Construction and preparation of $pTcosAp^r/ori/cm^r/neo^r$ ( Fig. 4A, B, C )

(a)Construction and preapration of pHScos:

(i) Construction

Cosmid vector pRH(-44)(10 μg) in a reaction medium (100 μl)[150 mM NaCl, 6 mM Tris-HCl buffer (pH 7.9), 6 mM $MgCl_2$, 100 μg/ml bovine serum albumin (BSA)] was digested with restriction endonuclease <u>Bam</u>HI (60 units)(New England Biolabs) for 2 hours. Reaction mixture was extracted with equal volume of water saturated phenol. Aqueous layer was concentrated with secondary butanol, added 0.1 vol. 3 M sodium acetate (pH7.5) and twice vol. ethanol therein to precipitate DNA,

- 13 -

which was washed with 95% ethanol, dried with vacuum by water suction-pump. Dried precipitate was dissolved in aqueous solution (7 µl, —1µg/µl)(hereinafter designates as 10t/0.1E) consisting of 10 mM Tris-HCl buffer (pH 8.0) and 0.1 mM EDTA.

The DNA solution (1 µl)(—1µg) digested with BamHI in a reaction medium (25 µl)[50 mM Tris-HCl (pH 7.2), 10 mM $MgSO_4$, 0.1 mM DTT, 50 µg/ml BSA, 80 µM dATP, 80 µM dCTP, 80 µM dGTP and 80 µM dTTP] was reacted with Klenow enzyme (2 units)(Boehringer Mannheim) at 22°C for 30 minutes to prepare blunt end double strand DNA fragment from cohesive end complimentary double strand DNA fragment. Reaction mixture was treated at 65°C for 10 minutes to denature Klenow enzyme.

The DNA solution thereof (10 µl, 0.4 µg) and SalI linker [d(pG-G-T-C-G-A-C-C)](2 µg) were ligated in a reaction medium (20 µl)[50 mM Tris-HCl (pH 7.4), 10 mM $MgCl_2$, 10 mM DTT, 1 mM spermidine, 1 mM ATP and 100 µg/ml BSA] with T4-DNA ligase (6 units) at 22°C for 6 hours. Thereafter the reaction mixture was extracted with phenol to obtain precipitated DNA.

The DNA precipitate was digested with restriction endonuclease SalI (1000 units)(Takara Shuzo Co.) in a reaction medium solution (600 µl)[150 mM NaCl, 6 mM Tris-HCl buffer (pH 7.9), 6 mM $MgCl_2$, 6 mM DTT, 100 µg/ml BSA] at 37°C for 4 hours. Resulting reaction mixture was electrophoresed on preparative agarose gel at 10 mA for 12 hours. Digested DNA fragments can be observed by means of ethidium bromide stain and long wave ultraviolet irradiation. DNA was extracted with five volume of solution [0.5 M $NH_4OAc$, 1 mM EDTA (pH 7.5) : aq. saturated phenol = 1 : 1] from the portion of the gels which contained the 3.89 Kbp DNA fragment comprising cos, ori and Ap$^r$. Eluate was concentrated with sec. butanol and 0.1 volume of 3 M sodium acetate and 2 volumes of ethanol were added thereto to precipitate DNA. The precipitate was incubated with T4-DNA ligase (6 units) in a reaction medium (20 µl) [50 mM Tris-HCl buffer (pH 7.4), 10 mM MgCl$_2$, 10 mM DTT, 1 mM spermidine, 1 mM ATP and 100 µg/ml BSA] at 12°C for 17.5 hours to close DNA fragment (3.89 Kbp) comprising cos, ori and Ap$^r$, which was extracted with phenol. DNA was obtained as ethanol precipitate which was further dissolved in 0.1 M Tris-HCl buffer (pH 7.5) (20 µl).

(ii) Transformation:

DNA solution thereof (10 µl) was mixed with competent cells of E. coli HB 101 (100 µl) [logarithmic growth phase cells of E. coli HB 101 treated with 0.1 M CaCl$_2$ solution at 0 - 4°C for 15 minutes were resuspended in 0.1 M CaCl$_2$/14% glycerol (1/10 volume of previous culture medium), and stored at -70°C] and incubated at 0°C for 10 minutes, subsequently at 37°C for 5 minutes to incorporate DNA into E. coli cells. L-broth (1% Bacto tryptone, 0.5% yeast extract, 0.5% NaCl and 0.1% glucose) (2 ml) was added therein and reciprocally shake cultured at 37°C for 1 hour. The cell suspension was spread on an L-plate (a plate added agar to 1% in L-broth) containing ampicillin (20 µg/ml), and cultured at 37°C for 24 - 48 hours to obtain ampicillin resistant clone.

(iii) Small scale culture and preparation plasmid DNA:

Ap$^r$ clone was grown in L-broth (2 ml, containing Ap 20 µg/ml) at 37°C for overnight with shaking. Cultured medium (1.5 ml) in Eppendorf tube was centrifuged to colllect cells which were resuspended in the extract solution [8% sucrose, 0.5% Triton X-100, 50 mM EDTA (pH 8.0) and 10 mM Tris-HCl

buffer (pH 8)] (0.35 ml). Lysozyme solution (25 µl) [10 mg/ml, 10 mM Tris-HCl (pH 8.0)] was mixed therewith and stood the tube in boiling water for 40 seconds. Immediately the tube was centrifuged at 4°C for 15 minutes at 21,000 r.p.m. to obtain supernatant solution which was treated with RNase A solution (20 mg/ml, 10t/0.1E) (0.5 µl) and incubated at 37°C for 1 hour to decompose RNA. Reaction mixture was deproteinated with phenol extraction to obtain DNA as ethanol precipitate which was dissolved in 10t/o.1E (20 µl) to obtain plasmid DNA solution.

(iv) Specifying plasmid DNA:

The plasmid DNA (1 µl or 2 µl) was digested with restriction endonuclease HindIII, SalI, PvuII and PstI and electrophoresed on 0.4 - 1.5% agarose gel [40 mM Tris-HCl buffer, 20 mM sodium acetate, 2 mM EDTA (pH 8.15): 10 mA, 12 - 24 hours]. Digested DNA fragment can be detected by ethidium bromide stain and long wave ultraviolet irradiation. Restriction endonuclease map of plasmid DNA was illustrated to specify plasmid DNA of each clone. Then E. coli cells having a plasmid, pHScos, in which DNA fragment (0.31 Kbp) between BamHI site and SalI site of pRH(-44) was deleted and SalI linker was inserted therebetween, were obtained (Fig. 4A).

(v) Large scale culture and preparation of plasmid DNA:

E. coli cells obtained in (iv) hereinabove was reciplocally shake cultured in TYG-P medium (1% Bacto tryptone, 0.1% yeast extract, 0.2% glucose, 0.1% $NH_4Cl$, 0.3% NaCl, 0.01% $Na_2SO_4$, 0.08% $MgCl_2 \cdot 6H_2O$, 1.52% $Na_2HPO_4 \cdot 12H_2O$ and 0.3% $KH_2PO_4$) (1 lit.) (containing ampicillin 20 µg/ml) at 37°C. Chloramphenicol was added upto 100 µg/ml concentration at the time showing an absorbancy of culture medium at 600 nm

- 16 -                          **0183571**

being 1.0, further reciprocally shake cultured at 37°C for
15 hours.  Cells were collected by centrifuge (6,000 r.p.m.,
10 min.) and resuspended in isotonic buffer solution (18 ml)
comprising 25% sucrose and 50 mM Tris-HCl buffer (pH 8.0).
Lysozyme solution [5 mg/ml, 0.25 M Tris-HCl buffer (pH 8.0)]
(3.6 ml) was added therein, gently mixed, stood at 0°C for 5
minutes, added 0.25 M EDTA solution (pH 8.0, 7.2 ml), again
gently mixed and stood at 0°C for 5 minutes.  Solution of
10% SDS (2.7 ml) and 5 M NaCl (8.1 ml) were added thereto,
gently mixed, stood at 0°C for overnight and centrifuged
(20,000 r.p.m. for 45 min.) to obtain cleared lysate (39 ml).
RNase solution (20 mg/ml, 10t/o.1E)(20 µl) was added to the
cleared lysate and incubated at 37°C for 1 hour to decompose
RNA.  Thereafter a mixture was subjected to phenol extraction
for deproteinization, and gel-filtered through Sepharose 2B.

DNA ethanol precipitate was obtained from void
fraction containing plasmid DNA and the DNA was subjected to
equilibrium density gradient centrifugation (60,000 r.p.m.,
12 hours) in cesium chloride—ethidium bromide solution [1M
Tris-HCl buffer (pH 8.0) 0.375 ml, 0.25 M EDTA (pH 8.0)
0.30 ml, $H_2O$ 6.45 ml, CsCl 17.5 g and EtBr solution (4 mg/ml)
0.375 ml].  According to this operation, covalently
closed  ,circular  plasmid DNA (ccc-DNA) can be detected, under
long wave ultraviolet irradiation, as zonal pattern below
the positions of fluorescent band of linear plasmid DNA and
chromosome in the centrifugal tube.  The ccc-DNA fraction
was collected, extracted with isopropanol saturated with
aqueous CsCl and dialyzed against 10t/o.1E (1 lit.) after
removal of ethidium bromide.  The ccc-DNA was precipitated
by adding ethanol in the dialysate.  Precipitate was dissolved

in 10t/o.1E (2 ml) to prepare pHS cos plasmid DNA solution.

(b) Construction and preparation of PHB cos:

A pHS cos (10 µg) in a reaction medium (400 µl) was digested with SalI (185 units) at 37°C for 2 hours. After deproteination with phenol extraction, precipitate was dissolved in 10t/0.1E (7 µl) (– 1 µg/µl). The SalI digested DNA solution (1 µl) (–1 µg) in a reaction medium (25 µl) was treated with Klenow enzyme (2 units) at 22°C for 30 minutes to convert from cohesive end to blunt end.

Klenow enzyme was denatured by heat treatment at 65°C for 10 minutes.

The said DNA solution (10 µl, 0.4 µg) and BamHI linker [d(pC-G-G-A-T-C-C-G)] (2 µg) in a reaction medium (20 µl) were ligated by     T4-DNA ligase (6 units) at 22°C for 6 hours.

The reaction mixture (10 µl) in a reaction medium (50 µl) was incubated with T4-DNA ligase (15 units) at 12°C for 17.5 hours to close the above ligate. Then the reaction mixture was extracted with phenol and added ethanol therein to precipitate DNA which was dissolved in 0.1 M Tris-HCl buffer (pH 7.5, 20 µl).

The thus obtained DNA solution (10 µl) was subjected to transformation into E. coli cells (HB 101) to obtain Ap$^r$ clone. Checking the specificity of plasmid in the said clone, a plasmid wherein BamHI linker was inserted in SalI site within pHS cos (in this case, SalI site was reconstructed within two distal BamHI site), designated as pHB cos in E. coli cells was obtained (Fig. 4A).

The thus obtained E. coli cells were grown in TYG-P medium (1 lit.) and isolated ccc-DNA from the cells to prepare a plasmid DNA, pHB cos.

(c)  Construction and preparation of pCH cos:

A cosmid vector pLH(+129)(10 µl) in a reaction medium (100 µl)[50 mM NaCl, 100 mM Tris-HCl buffer (pH 7.5), 5 mM MgCl$_2$, 100 µg/ml BSA] was digested with restriction endonuclease EcoRI (69 units)(Takara Shuzo Co.) at 37°C for 2 hours.

Reaction mixture was extracted with phenol for deproteination, and added ethanol therein to precipitate DNA which was dissolved in 10t/0.1E (7 µl)(—1 µg/µl).

A solution (1 µl)(—1 µg) of EcoRI digested DNA in a reaction medium (25 µl) was incubated with Klenow enzyme (2 units) at 22°C for 30 minutes for converting cohesive end to blunt end thereof.  Resulting reaction mixture was treated at 65°C for 10 minutes for denaturation of Klenow enzyme.

The obtained DNA solution (10 µl, 0.4 µg) and ClaI linker [d(pC-A-T-C-G-A-T-G)](2 µg) in a reaction medium (20 µl) were ligated         each other by treating with T4-DNA ligase (6 units) at 22°C for 6 hours.

Reaction mixture (10 µl) in a reaction medium (50 µl) was incubated with T4-DNA ligase (15 units) at 12°C for 17.5 hours to close DNA-fragment.  Resulted incubation mixture was extracted with phenol and added ethanol therein to precipitate DNA which was dissolved in 0.1 M Tris-HCl buffer (pH 7.5, 20 µl).

DNA solution (10 µl) was subjected to transformation in E. coli cells (HB 101) to obtain Ap$^r$ clone.  Checking the specificity of the said cloned plasmid DNA, E. coli containing pCH cos, a plasmid wherein ClaI linker was inserted in EcoRI site within pLH (+129)(in this case EcoRI site was reconstructed in two distal ClaI sites)(Fig. 4A) was obtained.

After growing the said E. coli in TYG-P medium (1 lit.), ccc-DNA was isolated and purified from the cells to prepare plasmid DNA of pCH cos.

(d) Construction and preparation of pCH/HB cos (pD cos):

A pCH cos (20 µg) in a reaction medium (200 µl) [60 mM NaCl, 7 mM Tris-HCl buffer (pH 7.4), 7 mM MgCl$_2$, 6 mM DTT and 100 µg/ml BSA] was digested with restriction endonuclease HindIII (114 units) (New England Biolabs) and PvuII (192 units) (Takara Shuzo Co.) at 37°C for 2 hours. Reaction mixture was electrophoresed on 0.8% preparative agarose gel. DNA fragment (2.5 Kbp) containing cos, Ap$^r$ and ori was extracted, then added ethanol therein to precipitate DNA which was dissolved in 10t/o.1E (8.5 µl) (~1 µg/µl).

On the other hand, pHB cos (20 µg) in a reaction medium (20 µl) was digested with HindIII (114 units) and PvuII (192 units) at 37°C for 2 hours. Reaction mixture was electrophoresed on 0.8% preparative agarose gel. Separated DNA fragment (1.6 Kbp) containing cos was extracted. Ethanol was added in the extract to precipitate DNA which was dissolved in 10t/0.1E (6.0 µl) (~1 µg/µl).

DNA solution (1 µl) (~1 µg), a digested fragment with HindIII/PvuII treatment, which contains cos, Ap$^r$ and ori in pCH cos, prepared in the former hereinabove, and DNA solution (1 µl) (~1 µg), a digested fragment with HindIII/PvuII treatment, which containes cos in pHB cos, prepared in the latter hereinabove, in a reaction medium (20 µl) were ligated by treating with T4-DNA ligase (6 units) at 12°C for 17.5 hours. Reaction mixture was extracted with phenol and DNA was obtained as ethanol precipitate, which was dissolved in 0.1 M Tris-HCl buffer (pH 7.5, 20 µl).

0183571

DNA solution (10 µl) was subjected to transformation into E. coli (HB 101) to obtain $Ap^r$ clone. Checking the specificity of plasmid DNA of the said clone, E. coli containing pDcos, a plasmid having two cos, $Ap^r$ and ori (Fig. 4A).

E. coli was grown in TYG-P medium (1 lit.), then ccc-DNA was isolated from cells and purified to prepare plasmid DNA of pDcos.

(e) Construction and preparation of $pHC-79/cm^r$:

A cosmid vector pHC-79 [Hohn and Collins, Gene, 11, 291 (1980)] (10 µg) in a reaction medium (100 µl) [50 mM NaCl, 6 mM Tris-HCl buffer (pH 7.9), 6 mM $MgCl_2$ and 100 µg/ml BSA] was digested with restriction endonuclease ClaI (Boehringer Mannheim) at 37°C for 2 hours. After deproteinization by phenol extraction, ethanol was added to precipitate DNA, which was dissolved in 10t/0.1E (7 µl) (—1 µg/µl).

On the other hand, pNT-31 (10 µg) in a reaction medium (200 µl) [100 mM NaCl, 10 mM Tris-HCl buffer (pH 8.4), 6 mM $MgCl_2$, 5 mM DTT and 100 µg/ml BSA] was digested with restriction endonuclease TaqI (40 units) (New England Biolabs) at 65°C for 2 hours. A reaction mixture was electrophoresed on 0.8% preparative agarose gel. DNA fragment (0.79 Kbp) containing $Cm^r$ was separated and purified, then added ethanol to precipitate DNA. The precipitate was dissolved in 10t/0.1E (3.0 µl) (—1 µg/µl).

DNA solution (1 µl) (—1 µg), digested pHC-79 with ClaI and DNA solution (1 µl) (—1 µg), a TaqI digested fragment of pNT-31 containing $cm^r$, in a reaction medium (20 µl) were treated with T4-DNA ligase to ligate each other at 12°C for 17.5 hours. Resulting mixture was extracted with phenol for deproteinization and DNA was precipitated by adding ethanol. Precipitate was dissolved in 0.1 M Tris-HCl buffer (pH 7.5)

DNA solution (10 µl) was subjected to transformation into E. coli cells (HB 101) to obtain cm$^r$ and Ap$^r$ clone. Checking the specificity of plasmid DNA of these clones, E. coli containing plasmid pHC-79/cm$^r$ wherein cm$^r$ was inserted into ClaI site within pHC-79 was obtained.

E. coli was grown in TYG-P medium (1 lit.), and ccc-DNA was isolated from cells and purified to prepare plasmid DNA of pHC-79/cm$^r$.

(f)Construction and preparation of pHC-79/cm$^r$ Δ (HindIII):

A plasmid pHC-79/cm$^r$ (10 µg) in a reaction medium (100 µl) was digested with HindIII (35 units) at 37°C for 2 hours. After deproteinization with phenol extraction, ethanol was added to obtain precipitated DNA, which was dissolved in 10t/0.1E (7 µl) (—1 µg/µl).

The digested DNA solution (1 µl) (—1 µg) in a reaction medium (25 µl) was treated with Klenow enzyme (2 units) at 22°C for 30 minutes to convert cohesive end thereof to blunt end. Reaction mixture was heated at 65°C for 10 minutes to denature Klenow enzyme.

The thus obtained DNA sclution (10 µl) (—0.4 µg) in a reaction medium (50 µl) was treated with T4-DNA ligase (5 units) at 12°C for 17.5 hours to close the DNA fragment. After phenol extraction, ethanol was added to precipitate DNA. Precipitated DNA was dissolved in 0.1 M Tris-HCl buffer (pH 7.5) (20 µl).

The DNA solution (10 µl) was subjected to transformation into E. coli cells (HB 101) to obtain Ap$^r$ and cm$^r$ clone. Checking the specificity of plasmid DNA of these clones, E. coli containing plasmid pHC-79/cm$^r$ Δ (HindIII) which defects HindIII site within pHC-79/cm$^r$. (Fig. 4B).

E. coli cells were grown in TYG-P medium (1 lit.), then ccc-DNA was isolated from cells and purified to prepare plasmid DNA of pHC-79/cm$^r$Δ(HindIII).

(g)Construction and preparation of pUC-8/cm$^r$:

A pHC-79/cm$^r$Δ(HindIII)(20 μl) in a reaction medium (200 μl) [50 mM (NH$_4$)$_2$SO$_4$, 20 mM Tris-HCl (pH 7.5), 10 mM MgCl$_2$ and 100 μg/mlBSA] was digested with restriction enzyme PstI (22 units, new England Biolabs) at 37°C for 2 hours. Further reaction mixture in a reaction medium (500 μl) was digested with BamHI (80 units) at 37°C for 2 hours and was electrophoresed on 0.8% preparative agarose gel, then DNA fragment (1.9 Kbp) containing cm$^r$ was extracted from the portion of the gel. DNA was precipitated by adding ethanol and the precipitate was dissolved in 10t/0.1E (4.0 μl)(−1 μl/μl).

On the other hand, pUC-8 [Jeffrey Vieira and Joachim Messing, Gene, 19, 259 (1982)](20 μg) in a reaction medium (200 μl) was digested with PstI (60 units) at 37°C for 2 hours, further digested in a reaction medium (500 μl) with BamHI (215 units) at 37°C for 2 hours. The digested DNA was electrophoresed on 0.8% preparative agarose gel. DNA fragment (2.7 Kbp) containing Ap$^r$ and ori was extracted from the portion of the gel. Ethanol was added to the extract to precipitate DNA which was dissolved in 10t/0.1E (14.0 μl)(−1 μg/μl).

DNA solution (1 μl)(−1 μg) of PstI/BamHI fragment containing cm$^r$ within pHC-79/cm$^r$Δ(HindIII) obtained in the former hereinabove and DNA solution (1 μl)(−1 μg) of PstI/BamHI fragment containing Ap$^r$ and ori within pUC-8 obtained in the latter hereinabove in a reaction medium (20 μl) were ligated each other with T4-DNA ligase (6 units) at 12°C for 17.5 hours. Thereafter the reaction mixture was extracted with phenol and ethanol was added therein to precipitate DNA.

The thus obtained DNA was dissolved in 0.1 M Tris-HCl buffer (pH 7.5, 20 µl).

The said DNA solution (10 µl) was subjected to transformation into E. coli cells (HB 101) to obtain cm$^r$ and Ap$^r$ clone. Checking the specificity of these clones, E. coli containing plasmid pUC-8/cm$^r$ having Ap$^r$, ori and cm$^r$. (Fig. 4B).

E. coli was grown in TYG-P medium (1 lit.) and ccc-DNA was isolated from the cultured cells then purified to prepare plasmid DNA of pUC-8/cm$^r$.

(h) Construction and preparation of pUC-8/cm$^r$ Δ (EcoRI/PstI):

pUC-8/cm$^r$ (20 µg) in a reaction medium (200 µl) was partially digested with EcoRI (60 units) at 37°C for 30 min. Digestate was extracted with phenol to deproteinate and added ethanol therein to obtain precipitated DNA. DNA precipitate was digested in a reaction medium (200 µl) with PstI (35 units) at 37°C for 2 hours. Again phenol extraction and ethanol precipitation were performed and precipitated DNA was dissolved in 10t/0.1E (10 µl) (—1 µg/µl). DNA solution (1.5 µl) (—1.5 µg) was treated in a reacion medium (30 µl) [33 mM Tris-acetate buffer (pH 7.9), 66 mM potassium acetate, 10 mM MgCl$_2$, 0.5 mM DTT, 100 µg/ml BSA, 0.1 mM dATP, 0.1 mM dCTP, 0.1 mM dGTP and 0.1 mM dTTP] with T4-DNA polymerase (5 units, P.L. Co.) at 37°C for 15 minutes for conversion of cohesive end thereof to blunt end, and was electrophoresed on 0.8% preparative agarose gel. DNA fragment (3.8 Kbp) containing Ap$^r$, ori and cm$^r$ was extracted from the portion of the gel. then DNA was obtained by ethanol precipitation.

Precipitated DNA was ligated in a reaction medium (50 µl) with T4-DNA ligase (15 units) at 12°C for 17.5 hours to close DNA fragment. After phenol extraction and ethanol precipitation, precipitated DNA was dissolved in 0.1 M Tris-HCl buffer (pH 7.5, 20 µl).

DNA solution (10 µl) was subjected to transformation into E. coli (HB 101) to obtain Ap$^r$ and cm$^r$ clone. Checking the specificity of plasmid DNA in these clones, E. coli having plasmid pUC-8/cm$^r$Δ(EcoRI/PstI) which deleted DNA fragment (0.75 Kbp) between EcoRI site and PstI site within pUC-8/cm$^r$. (Fig. 4B).

E. coli cells were grown in TYG-P medium (1 lit.) and ccc-DNA was isolated from the cells, then purified to obtain plasmid DNA, pUC-8/cm$^r$Δ(EcoRI/PstI).

(i)Construction and preparation of pUC-8/cm$^r$Δ(EcoRI/PstI)Δ(BamHI):

pUC-8/cm$^r$Δ(EcoRI/PstI)(10 µg) was digested in a reaction medium (100 µl) with BamHI (150 units) at 37°C for 2 hours. After subjecting the reaction mixture to phenol extraction and ethanol precipitation, precipitated DNA was dissolved in 10t/0.1E (7 µl)(−1 µg/µl).

The BamHI digested DNA solution (1 µl)(−1 µg) was treated in a reaction medium (25 µl) with Klenow enzyme (2 units) at 22°C for 30 minutes for converting cohesive end thereof to blunt end. Resulting reaction mixture was heated at 65°C for 10 minutes to denature Klenow enzyme. Reaction mixture (10 µl) was treated in a reaction medium (50 µl) with T4-DNA ligase (15 units) at 12°c for 17.5 hours to close DNA fragment. After subjecting to phenol extraction and ethanol precipitation, precipitated DNA was dissolved in Tris-HCl buffer (pH 7.5, 20 µl).

DNA solution (10 µl) was subjected to transformation into E. coli cells (HB 101) to obtain Ap$^r$ and cm$^r$ clone. ecking the specificity of plasmid DNA in these clones,

- 25 -

E. coli having plasmid: pUC-8/cm$^r$ Δ (EcoRI/PstI) Δ (BamHI) which defects BamHI site within pUC-8/cm$^r$ Δ (EcoRI/PstI) was obtained (Fig. 4B).

E. coli was grown in TYG-P medium (1 lit.), and ccc-DNA was separated from cells to prepare plasmid DNA pUC-8/cm$^r$ Δ (EcoRI/PstI) Δ (BamHI).

(j) Construction and preparation of pUC-8/cm$^r$ Δ (EcoRI/PstI) Δ (BamHI)/HindIII:

pUC-8/cm$^r$ Δ (EcoRI/PstI) Δ (BamHI) (100 µg) was digested in a reaction medium (100 µl) [20 mM KCl, 6 mM Tris-HCl buffer (pH 8.0), 6 mM MgCl$_2$, 6 mM DTT, 100 µg/ml BSA] with restriction endonuclease SmaI (125 units, New England Biolabs) at 37°C (blunt end). Reaction mixture was deproteinized by phenol extraction and DNA was precipitated by adding ethanol. Precipitate was dissolved in 10t/0.1E (7 µl) (—1 µg/µl).

The DNA solution (0.4 µl) (—0.4 µg) and HindIII linker [d(pC-A-A-G-C-T-T-G)] (2 µg) were ligated with T4-DNA ligase (6 units) at 22°C for 6 hours.

The said reaction mixture (10 µl) was treated in a reaction medium (50 µl) with T4-DNA ligase (15 units) at 12°C for 17.5 hours to close the molecule. After phenol extraction, DNA was obtained as ethanol precipitation and the precipitate was dissolved in 0.1 M Tris-HCl buffer (pH 7.5, 20 µl).

DNA solution was subjected to transformation into E. coli (HB 101) to obtain Ap$^r$ and cm$^r$ clone. Checking the specificity of these clones, E. coli having plasmid pUC-8/cm$^r$ Δ (EcoRI/PstI) Δ (BamHI)/HindIII in which HindIII linker was inserted into SmaI site of pUC-8/cm$^r$ Δ (EcoRI/PstI) Δ (BamHI) (Fig. 4A and Fig. 4B).

E. coli was grown in TYG-P medium (1 lit.) and isolated ccc-DNA from cells to purify pUC-8/cm$^r$ Δ (EcoRI/PstI) Δ (BamHI)/HindIII plasmid DNA.

(k) Construction and preparation of pDcos/cm$^r$:

A pDcos (10 µg) was digested in a reaction mixture (100 µl) with HindIII (60 units) at 37°C for 2 hours. After deproteinization by phenol extraction, ethanol precipitated DNA was obtained and was dissolved in 10t/0.1E (7 µl) (—1 µg/µl).

On the other hand, pUC-8/cm$^r$Δ(EcoRI/PstI)Δ(BamHI)/HindIII (20 µg) was digested in a reaction mixture (200 µl) with HindIII (25 units) at 37°C for 2 hours. Digestate was electrophoresed on 0.8% agarose gel to separate DNA fragment (1.15 Kbp) containing cm$^r$ which was obtained as ethanol precipitate. Precipitate was dissolved in 10t/0.1E (4.0 µl) (—1 µg/µl).

DNA solution (1 µl) (—1 µg) in which pDcos was digested with HindIII and DNA solution consisting of HindIII fragment which contain cm$^r$ in pUC-8/cm$^r$Δ(EcoRI/PstI)Δ(BamHI)/HindIII in a reaction medium (20 µl) were ligated to join each other with T4-DNA ligase (6 units) at 12°C for 17.5 hours. Reaction mixture was deproteinized by phenol extraction, and DNA was precipitated by adding ethanol. Precipitated DNA was dissolved in 0.1 M Tris-Hcl buffer (pH 7.5, 20 µl).

DNA solution (10 µl) was subjected to transformation into E. coli (HB 101) cells to obtain Ap$^r$ and cm$^r$ clone. Checking the specificity of these clones, E. coli containing plasmid pDcos/cm$^r$ having two cos, Ap$^r$, ori and cm$^r$ was obtained.

E. coli cells were grown in TYG-P medium (1 lit.) and isolated ccc-DNA from cells to prepare plasmid DNA of pDcos/cm$^r$.

(l) Construction and production of pHCcos:

A pRH(-44) (10 µg) was digested in a reaction mixture (100 µ) with PvuIII (23 units) at 37°C for 2 hours. Further reaction mixture was digested in a reaction medium (250 µl) with BamHI (70 units) at 37°C for 2 hours. Digestate was electrophoresed on 0.8% agarose gel to separate DNA fragment (2.48 Kbp) containing cos, Ap$^r$ and ori. DNA was precipitated by adding ethanol and was dissolved in 10t/0.1E (4.0 µl) (—1 µg/µl).

DNA solution (1 µl)(—1 µg) was treated in a reaction medium (25 µl) with Klenow enzyme (2 units) at 22°C for 30 minutes to convert cohesive end to blunt end. Reaction mixture was treated at 65°C for 10 minutes to denature Klenow enzyme.

The DNA solution (10 µl)(0.4 µg) and ClaI linker [d(pC-A-T-C-G-A-T-G)](2 µg) were ligated to join each other in a reaction medium with T4-DNA ligase (6 units) at 22°C for 6 hours.

Reaction mixture (10 µl) was treated in a reaction medium (50 µl) with T4-DNA ligase (15 units) at 12°C for 17.5 hours to close molecule. After phenol extraction, DNA was precipitated by adding ethanol and was dissolved in 0.1 M Tris-HCl buffer (pH 7.5)(20 µl).

Then DNA solution (10 µl) was subjecting to transformation into E. coli cells (HB101) to obtain Ap$^r$ clone. Checking the specificity of plasmid DNA in these clones, E. coli having plasmid pHCcos which defected DNA fragment (1.72 Kbp) between PvuII site and BamHI site in pRH(-44) and inserted ClaI linker therein (Fig. 4C).

E. coli was grown in TYG-P medium (1 lit.) and ccc-DNA was isolated from cells to prepare plasmid DNA of pHCcos.

(m)Construction and preparation of pBglIICcos:

A pHCcos (10 µg) was digested in a reaction medium (100 µl) with HindIII (100 units) at 37°C for 2 hours. After deproteinization by phenol extraction, DNA was precipitated by adding ethanol. Precipitated DNA was dissolved in 10t/0.1E (7 µl)(—1 µg) was treated in a reaction medium (25 µl) with Klenow enzyme (2 units) at 22°C for 30 minutes to convert cohesive end to blunt end. Reaction mixture was heated at 65°C for 10 minutes to denature Klenow enzyme.

The DNA solution (10 µl)(0.4 µg) and BglII linker [d(pC-A-G-A-T-C-T-G)](2 µg) were ligated to join each other by treating with T4-DNA ligase (6 units) at 22°C for 6 hours.

The reaction mixture (10 µl) was treated in a reaction medium (50 µl) with T4-DNA ligase (15 units) at 12°C for 17.5 hours to close the molecule. After phenol extraction, ethanol was added to precipitate DNA which was dissolved in 0.1 M Tris-HCl buffer (pH 7.5)(20 µl). The thus prepared DNA solution was subjected to transformation into E. coli (HB 101) to obtain Ap$^r$ clone. Checking the specificity of plasmid DNA in these clones, E. coli having plasmid pBglIICcos, in which BglII linker was inserted in HindIII site of pHCcos was obtained. (Fig. 4C).

E. coli was grown in TYG-P medium (1 lit.), then ccc-DNA was isolated from cells to prepare plasmid DNA pBglIICcos.

(n) Construction and preparation of pBR BamHI$^a$:

A pBR-327 (10 µg) was digested in a reaction medium (100 µl)[60 mM NaCl, 6 mM Tris-HCl buffer (pH 8.0), 10 mM MgCl$_2$, 6 mM DTT and 100 µg/ml BSA] with restriction endonuclease AvaI (55 units) at 37°C for 2 hours. After deproteinization with phenol extraction, DNA was obtained by adding ethanol, and was dissolved in 10t/0.1E (7 µl)(—1 µg/µl). DNA solution (1 µl)(—1 µg), which was digested with AvaI, was treated in a reaction medium (25 µl) with Klenow enzyme (2 units) at 22°C for 30 minutes for conversion of cohesive end thereof to blunt end. Reaction mixture was heated at 65°C for 10 minutes te denature Klenow enzyme.

The thus obtained DNA solution (10 µl, 0.4 µg) and BamHI linker [d(pC-G-G-A-T-C-C-G)] (2 µg) were ligated to join each other in a reaction medium (20 µl) with T4-DAN ligase (6 units) at 22°C for 6 hours. Thereafter phenol extraction was taken and DNA was obtained by adding ethanol.

The thus obtained DNA precipitate was digested in a reaction medium (600 µl) with BamHI (1,000 units) at 37°C for 4 hours, and electrophoresed on 0.8% preparative agarose gel to separate DNA fragment (2.22 Kbp) containing $Ap^r$ and ori, then DNA was obtained by ethanol precipitation.

The said DNA precipitate in a reaction medium (20 µl) was treated with T4-DNA ligase (6 units) at 12°C for 17.5 hours to close the molecule, then phenol extraction was taken and DNA was obtained by ethanol precipitation. Precipitated DNA was dissolved in 0.1 M Tris-HCl buffer (pH 7.5, 20 µl).

The thus obtained DNA solution (10 µl) was subjected to transformation into E. coli (HB 101) cells to obtain $Ap^r$ clone. Checking the specificity of plasmid DNA of these clones, E. coli containing plasmid pBR-327 $BamHI^a$, in which DNA fragment (1.05 Kbp) between BamHI site and AvaI site in pBR-327 was defected and BamHI linker was inserted therein, was obtained.

The said E. coli was grown in TYG-P medium (1 lit.) and ccc-DNA was separated from cells to prepare palsmid DNA pBR-327BamHI$^a$.

(o)Construction and preparation of pBR-327/BglIICcos:

A pBglIICcos (20 µg) was digested in a reaction medium (200 µl) with PstI (65 units) at 37°C for 2 hours, and was further digested in a reaction medium (500 µl) with ClaI

(77 units) at 37°C for 2 hours. Reaction mixture was electrophoresed on 0.8% agarose gel to separate DNA fragment (0.94Kbp) containing cos. DNA was precipitated by adding ethanol and the precipitate was dissolved in 10t/0.1E (50 μl)(—1 μg/μl).

On the other hand, pBR-327BamHI[a] (20 μg) was digested in a reaction medium (200 μl) with PstI (70 units) at 37°C for 2 hours, and was further digested in a reaction medium (500 μl) with ClaI (85 units) at 37°C for 2 hours. Reaction mixture was electrophoresed on 0.8% preparative agarose gel to separate DNA fragment (1.45 Kbp) containing ori. DNA was precipitated by adding ethanol and the precipitate was dissolved in 10t/10E (9.0 μl)(—1 μg/ l).

The DNA solution(1 μl)(—1 μg) of PstI/ClaI DNA fragment containing cos of pBglIICcos and a solution (1 μl)(—1 μg) of PstI/ClaI DNA fragment containing ori of pBR-327BamHI[a] were ligated to join each other in a reacion medium (20 μl) with T4-DNA ligase (6 units) at 12°C for 17.5 hours. Thereafter phenol extraction was taken and DNA was obtained as ethanol precipitate which was dissolved in Tris-HCl buffer (pH 7.5, 20 μl).

The DNA solution (10 μl) was subjected to transformation into E. coli (HB 101) cells to obtain Ap[r] clone. Checking the specificity of plasmid DNA of these clones, E. coli having a plasmid, pBR-327/BlgIICcos containing cos, Ap[r] and ori was obtained.

The E. coli was grown in TYG-P medium (1 lit.) and ccc-DNA was isolated from cells to prepare plasmid DNA pBR-327/BglIICcos.

(p)Construction and preparation of pTcos Ap[r]/ori/cm[r]:

A pDcos/cm[r] (20 μg) was digested in a reaction medium (200 µl) with ClaI (37 units) at 37°C for 2 hours, and resulting digestate was further digested in a reaction medium (500 µl) with BamHI (110 units) at 37°C for 2 hours. Reaction mixture was electrophoresed on 0.8% agarose gel to separate DNA fragment (1.5 Kbp) containing two cos and $cm^r$. DNA was obtained by ethanol precipitation which was dissolved in 10t/0.1E (4.0 µl) (—1 µg/µl).

On the other hand, pBR-327/BglIICcos (20 µg) was digested in a reaction medium (200 µl) with ClaI (80 units) at 37°C for 2 hours, and was further digested in a reaction medium (500 µl) with BamHI (240 units) at 37°C for 2 hours. DNA fragment (2.0 Kbp) containing cos, $Ap^r$ and ori was electrophoresed on 0.8% preparative agarose gel. Extracted DNA was precipitated by adding ethanol and the precipitate was dissolved in 10t/0.1E (12 µl) (—1 µg/µl).

DNA solution (1 µl) (—1 µg) of ClaI/BamHI fragment containing two cos and $cm^r$ in pDcos/$cm^r$ and DNA solution (1 µl) (—1 µg) of ClaI/BamHI fragment containing cos, $Ap^r$ and ori in pBR-327/BglIICcos were ligated to join each other with T4-DNA ligase (6 units) in a reaction medium (20 µl) at 12°C for 17.5 hours. Phenol extraction for deproteination and ethanol precipitation provided a DNA precipitate, which was dissolved in 0.1 M Tris-HCl buffer (pH 7.5, 20 µl).

The thus obtained DNA solution (10 µl) was subjected to transformation into E. coli (HB 101) cells to obtain $Ap^r$, $cm^r$ clone. Checking the specificity of plasmid DNA in these clones, E. coli containing plasmid pTcos $Ap^r$/ori/$cm^r$ having three cos, $Ap^r$, ori and $cm^r$ (Fig. 4).

The said E. coli was grown in TYG-P medium (1 lit.),

and ccc-DNA was separated from cells to prepare pTcos Ap$^r$/ori/cm$^r$ plasmid DNA.

(g)Construction and preparation of pSV010/neo$^r$:

A pAO43 [Oka, Sugisaki and Takanami, J. Mol. biol., 147, 214 (1981)](200 µg) was digested in a reaction medium (200 µl)[60 mM NaCl, 6 mM Tris-HCl buffer (pH 8.01), 10 mM MgCl$_2$, 6 mM DTT, 100 µg/ml BSA] with restriction endonuclease AvaII (New England Biolabs) at 37°C for 2 hours. Reaction mixture was electrophoresed on 0.8% preparative agarose gel to separate DNA fragment (1.24 Kbp) containing neo$^r$. DNA was obtained by adding ethanol and the precipitate was dissolved in 10t/0.1E (5 µg)(—1 µg/µl).

The said DNA solution (2 µl)(—2µg) was treated in a reaction medium (50 µl) with Klenow enzyme (4 units) at 22°C for 30 minutes for conversion of cohesive end thereof to blunt end. After deproteination by phenol extracion, DNA was precipitated by adding ethanol. Precipitated DNA was dissolved in 10t/0.1E (1.5 µl)(—1 µg/µl).

On the other hand, pSV010 {affinity plasmid of pSV01 [Tjan, R. et al., Proc. Natl. Acad. Sci., U.S.A., 77, 6491 91981)] which has polylinker originated from πVX [Brainseed, Nucleic Acids Res., 11, 2427 (1983)}(10 µg) was digested in a reaction medium (100 µl) with BamHI (129 units) at 37°C for 2 hours. Reaction mixture was deproteinized by phenol extraction and DNA was obtained by ethanol precipitation. Precipitate was dissolved in 10t/0.1E (7 µl)(—1 µg/µl).

Dna solution (2 µl)(—2 µg) was treated in a reaction medium (50 µl) with Klenow enzyme (4 units) at 22°C for 30 minutes to convert cohesive end thereof to blunt end. After phenol extraction for deproteination, DNA was obained by ethanol precipitation and was dissolved in 10t/0.1E (1.5 µl) (—1 µg/ µl).

DNA solution (1 µl) (—1 µg), in which DNA fragment containing neo$^r$ in pAO43 was treated with Klenow enzyme, and the DNA solution (1 µl) (—1 µg), which was prepared by Klenow enzyme treatment after BamHI digestion of pSV010, were ligated to join with T4-DNA ligase (6 units) at 12°C for 17.5 hours. Thereafter phenol extraction and ethanol precipitation were taken to obtain precipitated DNA which was dissolved in Tris-HCl buffer (pH 7.5, 20 µl).

The said DNA solution (10 µl) was subjected to transformation into E. coli (HB 101) to obtain Ap$^r$ and Km$^r$(neo$^r$) clone. Checking the specificity of the plasmid in these clones, E. coli having plasmid pSV010/neo$^r$ in which neo$^r$ was inserted in BamHI site of pSV010 (Fig. 4A).

E. coli was grown in TYG-P medium and ccc-DNA was isolated from cells to prepare plasmid DNA of pSV010/neo$^r$.
(r)Construction and preparation of pTcos Ap$^r$/ori/cm$^r$/neo$^r$:

A pTcos Ap$^r$/ori/cm$^r$ (10 µg) was partially digested in a reaction medium (100 µl) with EcoRI (27 units) at 37°C for 30 minutes. After deproteinization by phenol extraction, DNA was obtained by ethanol precipitation. Precipitated DNA was dissolved in 10t/0.1E (7 µl) (—1 µg/ µl).

On the other hand, pSV010/neo$^r$ (20 µg) was digested in a reaction medium (200 µl) with EcoRI (325 units) at 37°C for 2 hours. DNA fragment (1.3 Kbp) containing neo$^r$ was separated by electrophoresis on 0.8% preparative agarose gel. DNA was obtained by ethanol precipitation and was dissolved in 10t/0.1E (5.0 µl) (—1 µg/µl).

DNA solution (1 µl) (—1 µg), which was obtained by partially digestion of pTcos Ap$^r$/ori/cm$^r$ with EcoRI, and DNA solution (1 μl)(−1 μg) of EcoRI fragment containing neo$^r$ of pSV010/neo$^r$, were ligated to join each other with T4-DNA ligase (6 units) at 12°C for 17.5 hours.  Thereafter phenol extraction was taken and to obtain DNA by ethanol precipitation, then precipitate was dissolved in Tris-HCl buffer (pH 7.5, 20 μl).

The thus prepared DNA solution was subjected to transformation into E. coli (HB 101) cells and Ap$^r$, cm$^r$ and Km$^r$ (neo$^r$) clone was obtained.  Checking the specificity of plasmid DNA in these clones, E. coli having plasmid pTcos Ap$^r$/ori/cm$^r$/neo$^r$, in which neo$^r$ was inserted in EcoRI site of pTcos Ap$^r$/ori/cm$^r$, was obtained. (Fig. 1, Fig. 4A).

This strain of E. coli has been deposited in Fermentation Research Institute, Agency of Industrial Science and Technology, M.I.T.I., Japan as depostion No. FERM P-7888.

E. coli strain was grown in TYG-P medium (1 lit.) and ccc-DNA was isolated from cells to prepare plasmid DNA pTcos Ap$^r$/ori/cm$^r$/neo$^r$.

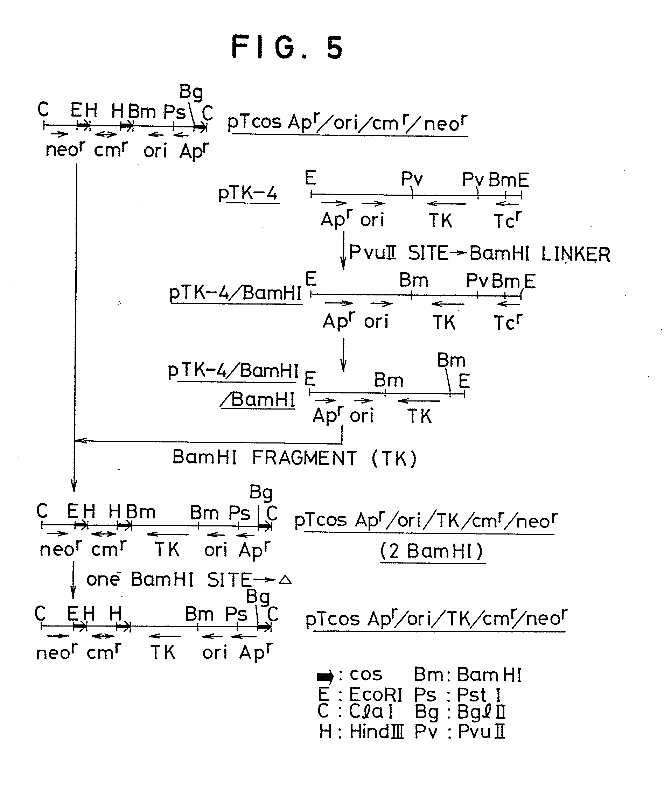

Example 2.
Construction and preparation of pTcos Ap$^r$/ori/TK/cm$^r$/neo$^r$: (Fig. 5) (a) Construction and preparation of pTK-4/BamHI:

A pTK-4 [Ishiura, M. et al. Mol. Cell. Biol., 2, 607 (1982)](10 μg) was partially digested in a reaction mixture (100 μl) with PvuII (3.0 units) at 37°C for 30 minutes. Reaction mixture was electrophoresed on 0.8% preparative agarose gel to separate 6.43 Kbp DNA fragment which was precipitated by adding ethanol.  Precipitated DNA was dissolved in 10t/0.1E (3 μl)(−1 μg/μl).

The thus prepared DNA solution (0.4 μl)(−0.4 μg) and

BamHI linker [d(pC-G-G-A-T-C-C-G)] (2 µg) were ligated to join each other in a reaction medium (20 µl) with T4-DNA ligase (6 units) at 22°C for 6 hours.

The resulting reaction mixture (10 µl) was treated in a reaction medium (50 µl) with T4-DNA ligase (15 units) at 12°C for 17.5 hours to close the molecule. Then reaction mixture was extracted with phenol and resulted DNA was precipitated by adding ethanol. Precipitate was dissolved in 0.1 M Tris-HCl buffer (pH 7.5, 20 µl).

The resulted DNA solution (10 µl) was subjected to transformation into E. coli (HB 101) cells to obtain $Ap^r$ and $Tc^r$ clone. Checking the specificity of the plasmid in these clones, E. coli having plasmid pTK-4/BamHI in which BamHI linker was inserted in PvuII site between ori and TK in pTK-4, was obtained. (Fig. 5)

E. coli was grown in TYG-P medium (1 lit.) and ccc-DNA was separated fromcells to prepare pTK-4/BamHI plasmid DNA.

(b) Construction and preparation of pTK-4/BamHI/BamHI:

A pTK-4/BamHI (10 µg) was limitedly digested in a reaction medium (100 µl) with BamHI (9 units) at 37°C for 30 minutes. Reaction mixture was extracted with phenol and added ethanol therein to precipitate DNA.

The ethanol precipitated DNA was digested in a reaction medium (100 µl) with PvuII (5 units) at 37°C for 2 hours, then the digestate was extracted with phenol and added ethanol therein to precipitate DNA, which was dissolved in 10t/0.1E (3 µl) (~1 µg/µl).

The thus obtained reaction mixture was limitedly digested with BamHI and further with PvuII. The DNA solution

(1 μl)(−1 μg) was reacted in a reaction medium (25 μl) with Klenow enzyme at 22°C for conversion of cohesive end thereof to blunt end. Resulting reaction mixture was electrophoresed on 0.8% preparative agarose gel to separated DNA fragment (4.67 Kbp) containing ori, Ap$^r$ and TK. DNA was obtained by adding ethanol.

The thus obtained precipitated DNA was religated with T4-DNA ligase (6 units) in a reaction mixture (20 μl) at 12°C for 17.5 hours to join two distal BamHI sites for closing. Resulting reaction mixture was extracted with phenol and added ethanol to precipitate DNA which was dissolved in 0.1 M Tris-HCl buffer (pH 7.5, 20 μl).

The said DNA solution (10 μl) was subjected to transformation into E. coli (HB 101) cells to obtain Ap$^r$ and Tc$^r$ clone. Checking the specificity of plasmid DNA in these clones, E. coli having plasmid pTK-4/BamHI/BamHI which defect DNA fragment (1.76 Kbp) between PvuII site and EAmHI site (on Tc$^r$) in pTK-4/BamHI. (Fig. 5)

The thus prepared E. coli was grown in TYG-P medium and ccc-DNA was separated from cells to prepare plasmid DNA of pTK-4/BamHI/BamHI.

(c) Construction and preparation of pTcos Ap$^r$/ori/TK/cm$^r$/neo$^r$(2 EamHI):

A pTcos Ap$^r$/ori/cm$^r$/neo$^r$ (10 μg) was digested in a reaction medium (100 μl) with BamHI (60 units) at 37°C for 2 hours. Digestate was extracted with phenol for deproteination and added ethanol therein to precipitate DNA which was dissolved in 10t/0.1E (7 μl) (−1 μg/μl).

On the other hand, pTK-4/BamHI/BamHI (20 μg) was digested in a reaction medium (200 μl) with BamHI (240 units) at 37°C for 2 hours. Resulting reaction mixture was electrophoresed on 0.8% preparative agarose gel to separate DNA fragment (2.0 Kbp) containing TK, then ethanol was added therein to precipitate DNA which was dissolved in 10t/0.1E (6.0 µl)(—1 µg/µl).

DNA solution (1 µl)(—1 µg) which was obtained by digestion of pTcos $Ap^r$/ori/$cm^r$/$neo^r$ with BamHI and BamHI DNA fragment solution (1 µl)(—1 µg) containing TK in pTK-4/BamHI/BamHI were ligated with T4-DNA ligase (6 units) at 12°C for 17.5 hours. Resulting solution was extracted with phenol and added ethanol to precipitate DNA which was dissolved in 0.1 M Tris-HCl buffer (pH 7.5, 20 µl).

The DNA solution (10 µl) was subjected to transformation into E. coli (HB 101) cells to obtain $Ap^r$, $cm^r$ and $Km^r$($neo^r$) clone. Checking the specificity of plasmid DNA in these clones, E. coli having plasmid pTcos $Ap^r$/ori/TK/$cm^r$/$neo^r$(2BamHI) in which TK was inserted in BamHI site of pTcos $Ap^r$/ori/$cm^r$/$neo^r$.

The said E. coli was grown in TYG-P medium (1 lit.) and ccc-DNA was separated from cells to prepare plasmid DNA; pTcos $Ap^r$/ori/TK/$cm^r$/$neo^r$ (2 BamHI).

(d) Construction and preparation of pTcos $Ap^r$/ori/TK/$cm^r$/$neo^r$:

ApTcos $Ap^r$/ori/TK/$cm^r$/$neo^r$ (2 BamHI)(10 µg) was partially digested in a reaction medium (100 µl) with BamHI (14 units) at 37°C for 30 minutes. Reaction mixture was extracted with phenol for deproteination and ethanol was added therein to precipitate DNA. Precipitate was dissolved in 10t/0.1E (7 µl)(—1 µg/µl).

DNA solution (1 µl)(—1 µg) which was obtained by partial digestion with BamHI, was treated in a reaction medium (25 µl) with Klenow enzyme (2 units) at 22°C for 30 minutes for conversion of cohesive end thereof to blunt end. Resulting DNA was electrophoresed on 0.8% preparative agarose gel to separate DNA fragment (6.93 Kbp) containing three cos, $Ap^r$, ori, TK, $cm^r$ and $neo^r$ and added ethanol therein to precipitate DNA.

The precipitated DNA was religated in a reaction medium (20 µl) with T4-DNA ligase (6 units) at 12°C for 17.5 hours to close the molecule. Resulted solution was extracted with phenol and ethanol was added therein to precipitate DNA which was dissolved in 0.1 M Tris-HCl buffer (pH 7.5, 20 µl).

The thus prepared DNA solution (10 µl) was subjected to transformation into E. coli (HB 101) cells to obtain $Ap^r$, $cm^r$ and $Km^r(neo^r)$ clone. Checking the specificity of plasmid DNA in these clones, E. coli having plasmid pTcos $Ap^r$/ori/TK/$cm^r$/$neo^r$ which defected BamHI site between $cm^r$ and TK in pTcos $Ap^r$/ori/TK/$cm^r$/$neo^r$ (2BamHI), was obtained. (Fig. 3 and 5).

The said E. coli strain was deposited in Fermentation Research Institute, Agency of Industrial Technology and Science, M.I.T.I., Japan, as permanent culture collection FERM P-7887.

The said E. coli was grown in TYG-P medium (1 lit.) and ccc-DNA was separated from cells to prepare plasmid DNA of pTcos $Ap^r$/ori/TK/$cm^r$/$neo^r$.

Example 3.

Preparation of gene bank for genomic DNA (extraneous DNA) with pTcos $Ap^r$/ori/$cm^r$/$neo^r$:

(a) Preparation of high molecular genomic DNA:

Mouse L-cell($LtK^-$[$HSV-1TK^+$]), in which a recombinant DNA of thymidine kinase (TK) of herpes symplex virus type I (HSV-1) having base arrangement of pBR-322 was inserted therein, was cultured in eagle MEM added with 10% calf serum to prepare confluent monolayers (100 mm plate; 9 plates, $-10^7$ cells/plate), and washed twice with PBS (137 mM NaCl, 3 mM KCl, 8 mM $Na_2HPO_4$, 1 mM $KH_2PO_4$). Solubilizer [100 µg/ml Proteinase K, 0.5% SDS, 150 mM NaCl, 10 mM EDTA, 10 mM Tris-HCl buffer (pH 7.5)] (1.0 ml) was added into a plate (1.0 ml/plate) to solubilize at 65°C for 15 - 30 minutes. The solubilized mixture was transferred to 50 ml centrifugal tube, further incubated at 37°C for overnight, and during the incubation Proteinase K (100 µg/ml) was added therein. Equal volume of phenol saturated with Tris-buffer was added therein to gently extract the solubilizate. Aqueous layer was dialysed for four times against dialysis buffer (3 lit.) consisting of 50 mM Tris-HCl buffer (pH 8.0), 10 mM EDTA and 10 mM NaCl. Dialysate was treated with adding 50 µg/ml DNase-free RNase A at 37°C for 3 hours to decompose RNA. Resulted solution was extracted twice with equal volume of a mixture of phenol and chloroform, and aqueous layer was dialysed for 9 times against aqueous solution (TE, 3 lit.) comprising 10 mM Tris-HCl (pH 8.0) and 1mM EDTA to obtain pruified DNA solution (50 ml).

Absorbancy (optical density, OD) of the DNA solution at 260 nm and 280 nm in 10 times dilution were 0.667 and 0.363 ($OD_{280}/OD_{260} = 0.544$), respectively. Concentration of DNA were 0.667 X 0.050 (µg/1OD) X 10 = 0.334 µg/µl.

(b) Preparation of 35 - 45 Kbp genomic DNA:

Purified genomic DNA solution (5.98 ml, 2 mg) obtained hereinabove was partially digested in a reaction medium (29.96 ml) [50 mM NaCl, 10 mM Tris-HCl buffer (pH 7.5), 10 mM $MgCl_2$ and 100 µg/ml BSA] with Sau3A (15.6 units) (New England Biolabs) at 37°C for 1 hour. 0.5 M EDTA (pH 8.0) solution (1.3 ml) was added therein and gently extracted twice with equal volume of Tris-HCl buffer saturated phenol. Aqueous layer was concentrated with sec. butanol upto 10 ml volume, then added 3.0 M sodium acetate (1.1 ml) and ethanol (20 ml) thereto, and added ethanol to precipitate genomic DNA which was washed with 70% ethanol. The ethanol precipitate was dried by means of section-pump and the pellet was dissolved in TE (2 ml).

The thus obtained DNA solution (300 µl) was subjected to 5 - 25% sucrose density gradient centrifuge [20 mM Tris-HCl buffer (pH 7.6), 5 mM EDTA, Beckmann SW 28, 20,000 r.p.m. for 9 hours at 20°C] and fractions with each 30 drops were obtained. Each fraction (20 µl) was electrophores· i on 0.4% agarose gel and measured the length thereof, collected 35 - 45 Kbp DNA fractions, then dialysed three times against TE (3 lit.). Dialysate was extracted with phenol and aqueous layer was concentrated with sec. butanol. DNA was obtained by adding ethanol, and the precipitate was dissolved in TE (1 ml) to obtain 35 - 45 Kbp genomic DNA. Absorbancy of DNA solution at 260 nm and 280 nm were $OD_{260}$ = 0.216 and $OD_{280}$ = 0.112 ($OD_{280}/OD_{260}$ = 0.519) in 50 times dilution. Concentration of DNA was 0.216 X 0.050 (µg/1OD) X 50 = 0.54 µg/µl.

(c) Preparation of cosmid vector DNA:

A pTcos $Ap^r/ori/cm^r/neo^r$ (40 µg) was digested in a reaction medium (400 µl) with $\underline{ClaI}$ (80 units) at 37°C for 2·hours. Reaction mixture was extracted with phenol and added ethanol therein to precipitate DNA which was dissolved in 10t/0.1E (100 µl).

The said $\underline{ClaI}$ digested DNA solution (95 µl) was treated in a reaction medium (500 µl)[50 mM Tris-HCl buffer (pH 9.0), 1 mM $MgCl_2$, 0.1 mM $ZnCl_2$ and 1 mM spermidine] with phosphatase (6 units)(calf intestinal alkaline phosphatase, Boehringer Mannheim) at 37°C for 15 minutes and at 56°C for 15 minutes. Further the treated reaction mixture was treated with adding phosphatase (6 units) at 37°C for 15 minutes, subsequently at 56°C for 15 minutes to inactivate ClaI fragment.

To a raction mixture thereof were added $H_2O$ (400 µl), STE [100 mM Tris-HCl buffer (pH 8.0), 1M NaCl and 10 mM EDTA] (100 µl) and 10% SDS (50 µl) and heated at 68°C for 15 minutes to denature phosphatase. The mixture was extracted with phenol for deproteinization and ethanol was added therein to precipitate DNA which was dissolved in 10t/o.1E (30 µl).

The resulting DNA solution (30 µl), which had digested with ClaI and denatured with phosphatase, was digested in a reaction mixture (500 µl) with BamHI (176 units) at 37°C for 2 hours. Then the mixture was extracted with phenol for deproteination and ethanol was added to precipitate DNA which was dissolved in 10t/0.1E (30 µl) to obtain cosmid vector DNA solution (0.69 µg/µl).

(d) Ligation of 35 - 45 Kbp genomic DNA and cosmid vector:

Genomic DNA (35 - 45 Kbp, 2.61 µl, 1.4 µg) prepared in (b) hereinabove and cosmid vector DNA (0.86 µl, 0.59 µg) prepared in (c) hereinabove were ligated in a reaction medium (10 µl) with T4-DNA ligase (5.9 units) at 12°C for 17.5 hours to join each other. Resulting reaction mixture was kept at 4°C.

(e) Preparation of ultrasonicated cell extracts from BHB 2690:

E. coli BHB 2690 was cultured in NZY-medium (1% NZ amine, 0.5% yeast extract, 0.5% NaCl and 0.2% $MgCl_2 \cdot 6H_2$), pH 7.5)(100 ml) for overnight. Absorbancy at 600 nm $(OD_{600})$ of cultured broth was measured and the said cultured medium was inoculated in an NZ medium (500 ml in 1 lit. flask), in

which $OD_{600}$ thereof was adjusted to 0.025 by inculating cells, and shake cultured at 32°C. After starting the cultivation for 2 hours 40 minutes, at the time for $OD_{600}$ was shown the value of 0.3, prophage was induced at 45°C for 15 minutes intervals. Culturing temperature was changed to set at 38 - 39°C and vigorously shake cultured for 3 hours. Induction was checked by adding a drop of chloroform to a small amount of cultured broth.

Cultured broth was centrifuged at 6,000 r.p.m. for 10 minutes to collect bacterial cells, which were suspended in a buffer solution [20 mM Tris-HCl (pH 8.0), 1 mM EDTA and 5 mM β-mercapto ethanol] (3.6 ml). Suspension was ultrasonicated, by maximum power for 5 seconds for 20 times, below 4°C, then centrifuged at 20,000 r.p.m. for 10 minutes at 4°C to obtain the supernatant solution (-3 ml). To a supernatant solution (3 ml) were added equal volume of buffer solution hereinabove and packaging buffer solution [6 mM Tris-HCl buffer (pH 8.0), 50 mM spermidine, 50 mM putrecin, 20 mM $MgCl_2$, 30 mM ATP and 30 mM β-mercaptoethanol] (0.5 ml), and each 15 µl thereof was injected into 1.5 ml Eppendorm tube and simultaneously freezed in a liquid nitrogen to keep at -70°C.

(ii) Preparation of cell extract by freezing thawing for BHB2688:

E. coli 2688 was cultured in NZY-medium (100 ml) for overnight. Absorbancy ($OD_{600}$) of cultured broth was measured at 600 nm, and the cultured broth was inoculated in a previously kept NZY-medium (500 ml in 2 lit. flask) at 32°C with adjusting $OD_{600}$ thereof to 0.025 and shake cultured at 32°C.

After starting cultivation at about 2 hours 40 minutes, at the time fo $OD_{600}$ reached 0.3, prophage was induced at 45°C for each 15 minutes intervals.  Culturing temperature thereof was changed to 38 - 39°C and a medium was vigorously shake cultured.  Induction was checked by dropping one drop of chloroform in a small volume of cultured broth.

Cultured broth was centrifuged at 6,000 r.p.m. for 10 minutes to collect cells and suspended in sucrose solution [10% sucrose, 50 mM Tris-HCl (pH 8.0)](3 ml), then each 0.5 ml thereof was injected into Eppendrof tube (6 tubes).  Lysozyme solution (2 mg/ml, 0.25 M Tris-HCl buffer (pH 8.0)](25 µl) was added therein at 4°C and gently mixed, then freezed in liquid nitrogen.

Each tube was set up on an ice to thaw the extract, and packaging buffer (25 µl) was added therein to mic solution.  The extracts were combined and centrifuged at 21,000 r.p.m. for 1 hour at 4°C to obtain supernatant, then each 10 µl thereof, respectively, was injected into 1.5 ml Eppendorf tube, and immediately freezed ina liquid nitrogen, then stored at -70°C.

(f) In vitro packaging:

Freezed extract of strain BHB 2690 and BHB 2688 stored at -70°C were put on an ice.  The strain BHB 2688 extract which was prior thawed was added to the BHB 2690 extract and gently mixed.  At about almost thawing the freezed extract, recombinant DNA solution (2.5 µl, 0.5 µg) prepared in (d) hereinabove was added therein to mxi well, and incubated at room temperature for 1 hour to perform in vitro packaging.

The reaction mixture thereof were added SM solution [0.58% NaCl, 0.2% $MgSO_4 \cdot 7H_2$), 50 mM Tris-HCl buffer (pH 7.5) and 0.01% gelatine](1 ml) and one drop of chlorform.

The mixture in Eppendorf tubes were centrifuged for 30 seconds to obtain a supernatant, transducing phage particle solution.

(g) Transduction and plating:

E. coli HB 101 was cultured in L-broth (added maltose 0.4%) for overnight. Cultured broth (200 μl) was cnetrifuged to collect cells, which was resuspended in 10 mM $MgCl_2$ solution (100 μl). Transducing phage particles solution (100 μl) diluted for 10 times with SM solution was added in teh cell suspension (100 μl) and incubated at 37°C for 15 minutes to transduce, then added L-broth (1.1 ml) therein to shake culture at 37°C for 45 minutes. Cell suspension was spread each 100 μl, respectively, on the 100 mm L-plate containing ampicillin 20 μg/ml, then incubated at 37°C for overnight. Colonies formed were picked up by bamboo skewer, which was grown in L-broth containing ampicillin 20 μg/ml.

Numbers of colony per plate were 20 - 100.

(h) culturing transformant, and preparation and specifying DNA thereof:

Ampicillin resistant colonies (transformant)(20 colonies) obtained in (g) hereinabove were cultured in L-broth (containing ampicillin 20 μg/ml)(2 ml) for overnight, and each cultured broth was injected into 1.5 ml Eppendorf tube which was centrifuged to collect cells.

Resuspended cells in the solution I [50 mM glucose, 25 mM Tris-HCl buffer (pH 8.0), 10 mM EDTA and 4 mg/ml lysozyme](100 μl) were stood at room temperature for 5 minutes. The solution II (0.2 N NaOH and 1% SDS)(200 μl) was added therein to mix gently and on an ice for 5 minutes. The solution III [5 M potassium acetate (pH 4.8)](150 μl) was added therein to mix gently, and laid on an ice for 5 minutes. A solution in Eppendorf tubes was centrifuged to

Ethanol (twice volume thereof) was added therein to precipitate plasmid DNA.

Plasmid DNA of each transformant was dissolved in a small amount of 10t/0.1E, which was electrophoresed on 0.4% agarose gel together with size-marker DNA ($\lambda$-DNA: 48 Kbp and digest of $\lambda$-DNA with HindIII: 22.3 Kbp and 9.5 Kbp). Among the tested 20 ampicillin resistant colonies, all the colonies showed the pattern of plasmid DNA on 22.3 Kbp - 48 Kbp in size.

In this stage, recombinant DNA exists as a plasmid, and an extraneous DNA gene bank is produced as plasmid DNA.

(i) Transduction and in vivo packaging:

A strain LN 900 [HB 101 ($\lambda$CI$^{ts}$, TC$^{r}$): $\lambda$-phage; lysogenization at 37°C; $\lambda$-phage; entering lytic cycle at 37°C. tetracycline resistance] in which $\lambda$-phage was lysogenized in E. coli HB 101, was cultured in L-broth (added maltose 0.4%) at 30°C for overnight. The cultured broth (200 µl) was resuspended in 10 mM MgCl$_2$ solution (100 µl). To the said suspension (100 µl) was added transducing $\lambda$-phage particles (diluted 10 times volume in SM solution) prepared in (f) hereinabove, and incubated at 30°C for 15 minutes for transduction. Further L-broth (1.1 ml) was added therein and shake cultured at 30°C for 45 minutes. Ampicillin and chloramphenicol were added to 20 µg/ml, then total volume 2 ml of the solution (L-broth; added maltose 0.4%) was incubated at 30°C for overnight.

The incubated medium (25 µl) was inoculated to L-broth (added maltose 0/4%, ampicillin 20 µg/ml and chloramphenicol 20 µg/ml)(5 ml), previously kept at 30°C, and shake cultured at 30°C.

After 3 hours from starting cultivation, at the time of $OD_{66}$ reaching at 0.3, the medium was subjected to induction at 40°C with 10 minutes intervals, and further shake cultured at 37°C for 30 minutes.  L-broth (added maltose 0.4%)(9.9 ml), previously kept at 37°C, was added to the cultured broth (100 µl), then shake cultured at 37°C for 2 hours., and added few drops of chloroform therein to mix the solution. The solution was cnetrifuged at 4,500 r.p.m. for 10 minutes to obtain the supernatant, a transducing phage particles solution.

To the transducing phage particles solution (50 µl) was added in cultured broth (1 ml) of E. coli LN 900, previously cultured at 30°C for overnight, and kept at 30°C for 5 minutes for transduction.  Cell suspension, each 50 µl, was spread on L-plate (containing ampicillin 20 µg/ml), then the plates were incubated at 30°C for overnight.  1000 - 3000 colonies per plate were observed.

In this stage, recombinant DNA exists as transducing phage particles and extraneous DNA gene bank is produced as transducing phage particles.

4. Brief explanation of the drawings:

Fig. 1: a novel cosmid vector pTcos $Ap^r$/ori/$cm^r$/$neo^r$;

Fig. 2: a process for preparing genomic DNA gene bank using novel cosmid vector of Fig. 1;

Fig. 3: a novel cosmid vector pTcos $Ap^r$/ori/TK/$cm^r$/$neo^r$;

Fig. 4A, 4B and 4C; a construction / method for pTcos $Ap^r$/ori/$cm^r$/$neo^r$; and

Fig. 5: a construction / method for pTcos $Ap^r$/ori/TK/$cm^r$/$neo^r$.

CLAIMS

1. A cosmid vector comprising at least three cos of λ-phage or lambdoid phage in tandem.

2. A cosmid vector according to claim 1 which contains a respective cleavage site for each of two restriction endonucleases such that, on digestion of the cosmid vector with the said restriction endonucleases, the cosmid vector is cleaved into left and right arms wherein one arm comprises at least one cos and the other arm comprises two cos.

3. A cosmid vector according to claim 2, wherein the arms are of approximately equal length.

4. A cosmid vector according to claim 2 or 3, wherein the right arm comprises at least one cos and the left arm comprises two cos.

5. A cosmid vector according to any one of the preceding claims, which comprises three cos.

6. A cosmid vector according to any one of the preceding claims, which comprises a marker gene coding for the expression of drug resistance or for a specific physiological function.

7. A cosmid vector according to claim 6, wherein the drug resistance is ampicillin resistance, tetracycline resistance, neomycin resistance or chloramphenicol resistance.

8. A cosmid vector according to claim 6, wherein the said physiological function is thymidine kinase (TK) activity, adenine phosphoribosyl transferase (APRT) activity, hypoxanthine guanine phosphoribosyl transferase (HGPRT) activity, xanthine guanine phosphoribosyl transferase (XGPRT or gpt) activity or dihydrofolic acid reductase (dhfr) activity.

9. A cosmid vector according to any one of the preceding claims, which comprises a gene region having a replication function originating from ColEI.

10. A process for preparing a gene bank which process comprises:

0183571.

- 48 -

(i)   cleaving a cosmid vector as claimed in any one of the preceding claims into left and right arms by digesting the cosmid vector with two restriction endonucleases, one arm having at least one cos and the other having two cos;

(ii)   ligating extraneous DNA fragments of 35 - 45 Kbp between respective pairs of left and right arms;

(iii)   packaging the recombinant DNA thus prepared as transducing phage particles by means of $\underline{in\ vitro}$ packaging and infecting $\lambda$-phage lysogenic $\underline{Escherichia\ coli}$ with the said particles; and

(iv)   after cloning, inducting an $\underline{E.\ coli}$ transformant to package the recombinant DNA contained therein as transducing phage particles.

11.   A process according to claim 10, wherein the extraneous DNA is a DNA from a microorganism, animal or plant.

12.   A drug-resistant cosmid vector comprising two cos of $\lambda$-phage or lambdoid phage in tandem and containing a respective cleavage site for each of the two restriction endonucleases such that, on digestion of the cosmid vector with the said restriction endonucleases, the cosmid vector is cleaved into two arms each having one cos.

13.   A microorganism transformed with a cosmid vector as claimed in any one of claims 1 to 9.

14.   Use of a cosmid vector as claimed in any one of claims 1 to 9 in the preparation of a gene bank by cosmid cloning.

15.   $\underline{Escherichia\ coli}$ HB101-pTcos Ap$^r$/ori/cm$^r$/neo$^r$.

16.   $\underline{Escherichia\ coli}$ HB101-pTcos Ap$^r$/ori/TK/cm$^r$/neo$^r$

17.   $\underline{Escherichia\ coli}$ HB101-pD cos/cm$^r$.

FIG. 1

cos : λ−PHAGE cos

ori : REPLICATION ORIGIN

$Ap^r$ : AMPICILLIN RESISTANT REGION

$neo^r$ : NEOMYCIN RESISTANT REGION

$cm^r$ : CHLORAMPHENICOL RESISTANT REGION

# FIG. 2

GENOME DNA (EXOGENOUS DNA)

ClaI

cos

neo$^r$

Ap$^r$ cos

ori cm$^r$

cos

BamHI

$\dfrac{pTcos\,Ap^r/ori/}{cm^r/neo^r}$

| ClaI DIGESTION | Sau 3A(OR MboI) PARTIAL DIGESTION |

neo$^r$ cm$^r$ ori Ap$^r$

cos cos cos

phosphatase (INACTIVATION)

* ⟶ ⟶ ⟶ *

Bam HI DIGESTION

* ⟶ ⟶ ⟶ *

Fractionation

35−45Kbp FRAGMENT (GENOME FRAGMENT)

T 4 DNA LIGASE

neo$^r$ cm$^r$ ori Ap$^r$

* ⟶ ⟶ ⟶ *

cos cos (GENOME FRAGMENT) cos

1) in vitro PACKAGING
2) INFECTION INTO E.coli
3) SELECTION BY AMPICILLIN−AND CHLORAMPHENICOL

cm$^r$

Ap$^r$ cos cos

ori

Genomic DNA library in plasmid DNA

1) induction
2) in vivo PACKAGING

Genomic DNA library in transducing phage particles

# FIG. 3

cos : λ-PHAGE cos

ori : REPLICATION ORIGIN

Apʳ : AMPICILLIN RESISTANT REGION

neoʳ : NEOMYCIN RESISTANT REGION

cmʳ : CHLORAMPHENICOL RESISTANT REGION

T K : TYMIDINE KINASE ACTIVITY REGION

# FIG. 4 A

```
              Sa
        H Bm⁄  Pv    Ps H                    E H    Pv    Ps H
pRH(-44) ├┤ ╱──┼────┼──┤                    ├┤──────┼────┼──┤  pLH(+129)
               ←   ←                              ←   ←
              ori  Apr                            ori  Apr

        │ △(Bam HI / SaℓI)                    │ Eco RI SITE
        │  → SaℓI LINKER                      │  → CℓaI LINKER

        H Sa  Pv   Ps H              C H     Pv   Ps E
pHScos  ├┤──┼───┼───┤            ╪╪═──────┼───┼──┤   pCHcos
              ←   ←              E        ←   ←
             ori  Apr                    ori  Apr

        │ SaℓI SITE
        │  → Bam HI LINKER

        H Bm Pv   Ps H                    PvⅡ/HindⅢ FRAGMENT
pHBcos  ├┤─┼──┼───┤                        (Apr/ori/cos)
         ╲Sa ori  Apr
        │ PvuⅡ/HindⅢ FRAGMENT(cos)
```

```
        E╲H Bm Pv    Ps E              H    H         H      *pUC-8/cmr
pDcos   ├┤┼┼─Sa┼────┼──┤              ├───┼────────┼──┤    △(PstI/EcoRI)
        C       ←   ←                      →      ←  ←      △(Bam HI)
               ori  Apr                   cmr   ori  Apr    /HindⅢ
                                     HindⅢ FRAGMENT(cmr)
```

```
            E  H   H Bm Sa Pv    PsE        C BmPs Bg    **pBR-327/BgℓⅡCcos
pDcos/cmr  ├┤──┼──┼┼─┼──┼────┼──┤          ├─┼──┼─┼─┤
           C  cmr      ori   Apr           H  ori  Apr
              ClaI/BamHI FRAGMENT
               (cos/cmr/cos)
                                    ClaI/BamHI FRAGMENT(Apr/ori/cos)
```

```
              Sa Ps
        E H   H Bm  Bg C            E     E        E   pSV010/neor
        ├┤─┼──┼┼──┼──┤             ├─────┼───────┼──┤
        C  cmr  ori  Apr                →      ←  ←
pTcos Apr/ori/cmr                       neor   ori  Apr
                              Eco RI FRAGMENT(neor)
```

```
                  Sa
        E   E H  H Bm Ps Bg C
        ├───┼──┼──┼┼─┼──┼──┤     pTcos Apr/ori/cmr/neor
        C  neor  cmr  ori  Apr
```

```
➡ : cos      Ps : Pst I
E  : EcoRI    Sa : SaℓI
Bm : BamHI    C  : CℓaI
Pv : Pvu Ⅱ    H  : HindⅢ
Bg : BgℓⅡ
```

0183571

# FIG. 4 B

➡ : cos    Ps : Pst I
E : EcoRI    T : Taq I
Bm:BamHI    Sm:Smal
C : Clal    H : HindⅢ

0183571

6/7

# FIG. 4 C

→ : cos          Ps : Pst I
E : EcoRI      Bm: BamHI
H : HindⅢ     A : Ava I
Pv: PvuⅡ      C : Cla I
Bg: BglⅡ

# FIG. 5

```
        Bg
C  EH  HBm Ps\C    pTcos Apr/ori/cmr/neor
├──┼┼──┼┼┼──┼─┤
 ──▶ ─▶ ◀── ─▶
neor cmr  ori Apr
```

```
                E          Pv      Pv BmE
        pTK-4    ├──────────┼───────┼─┼─┤
                 ──▶        ◀──     ◀──
                Apr ori      TK      Tcr
```

↓ PvuⅡ SITE → BamHI LINKER

```
                      E         Bm     Pv BmE
pTK-4/BamHI           ├─────────┼──────┼─┼─┤
                       ──▶ ─▶   ◀──    ◀──
                      Apr ori    TK     Tcr
```

↓

```
pTK-4/BamHI   E          Bm      Bm
                                  \E
/BamHI        ├──────────┼───────┼─┤
               ──▶ ─▶     ◀──
              Apr ori      TK
```

BamHI FRAGMENT (TK)

```
                            Bg
C  EH  HBm     Bm Ps │ C    pTcos Apr/ori/TK/cmr/neor
├──┼┼──┼┼──────┼──┼─┼─┤
 ──▶ ─▶ ◀──    ◀── ◀── ─▶        (2 BamHI)
neor cmr  TK   ori Apr
```

↓ one BamHI SITE → △

```
                         Bg
C  EH  H       Bm Ps \ C    pTcos Apr/ori/TK/cmr/neor
├──┼┼──┼───────┼──┼─┼─┤
 ──▶ ─▶ ─▶     ◀── ◀── ─▶
neor cmr  TK   ori Apr
```

```
━▶ : cos      Bm : BamHI
E : EcoRI     Ps : Pst I
C : ClaI      Bg : BglⅡ
H : HindⅢ     Pv : PvuⅡ
```